# EUROPEAN PATENT APPLICATION

(11) **EP 1 547 620 A2**
(43) Date of publication of application: **29.06.2005**
(21) Application number: 04257928.4
(22) Date of filing: 17.12.2004
(51) Int. Cl.: A61L 2/18, A61L 2/24, A01N 35/04

(54) **Germicidal compositions comprising phthalaldehyde**

(30) Priority: 19.12.2003 US 741525
(71) Applicant: ETHICON, INC., Somerville, NJ 08876 (US)
(72) Inventor: Wu, Su-Syin S., Irvine, CA 92614 (US); Chen, Xiaolan, Irvine, CA 92606 (US); Chan-Myers, Harriet, Lake Forest, CA 92630 (US); Choperena, Alfredo M., San Juan Capistrano, CA 92675 (US); Zhu, Peter C., Irvine, CA 92604 (US); Roberts, Charles G., Long Beach, CA 90815 (US); Herruzo, Rafael, 28760 Madrid (ES); Tran, Yvonne, Murrieta, CA 92562 (US)
(74) Representative: Fisher, Adrian John

(57) **Abstract**

Disclosed herein are germicidal compositions, kits, methods for preparing the germicidal compositions, and methods of using the compositions for disinfection or sterilization. In one aspect, a solid composition may include a solid having water solubility greater than phthalaldehyde, and a germicide diluted in the solid. In another aspect, a kit for preparing a germicide solution may include a germicide, an enhancer for the germicide, and at least two compartments to contain the germicide and the enhancer. In yet another aspect, an apparatus may be used to prepare a germicidal solution. The apparatus may include a first port to receive a first composition including a germicidally effective compound, a second port to receive a second composition including an enhancer for the germicidally effective compound, a source of water, and germicidal solution preparation logic to control preparation of the germicidal solution from the first and the second compositions and the water.

## Description

### CROSS-REFERENCE TO RELATED APPLICATIONS

The present application is related to the co-filed, co-pending, and coassigned case entitled "EFFICACY ENHANCERS FOR GERMICIDES" having U.S. Patent Application Serial No. 10/741529. This related patent application is hereby incorporated by reference.

### BACKGROUND

### Field

Embodiments of the invention relate to germicidal compositions, kits and methods for preparing the germicidal compositions, and methods of using the compositions for disinfection or sterilization.

### Background Information

Various aldehyde-based germicidal compositions are known in commerce and have been discussed in the literature. Among the more prevalent of the aldehyde-based germicidal compositions are those including formaldehyde, glutaraldehyde, or o-phthalaldehyde (also known simply as phthalaldehyde). Phthalaldehyde has certain advantages over formaldehyde and glutaraldehyde. Formaldehyde is potentially carcinogenic and has an objectionable odor. Glutaraldehyde likewise has an objectionable odor, and may be chemically unstable during storage. Phthalaldehyde is generally not regarded to be carcinogenic, is substantially odorless, and has rapid germicidal action. Due to these and other advantages, there is a general need in the arts for new and improved germicidal compositions containing phthalaldehyde.

One meter for measuring the performance of germicides is their ability to kill spores. U.S. Pat. No. 4,971,999, issued November 20, 1990, to Bruckner et al., discloses in part odorless sterilizing and disinfecting solutions that contain phthalaldehyde. The solutions are reported to have sporicidal activity against *Bacillus subtilis* and *Clostridium sporogenes* spores. As reported therein, a composition containing a low concentration of phthalaldehyde (e.g., 0.25%) as the sole active ingredient has sporicidal activity against *Bacillus subtilis* and *Clostridium sporogenes* spores in 24 hours at a temperature of 20°C. At higher concentrations (e.g., 1.0%) of phthalaldehyde, sterilization is achieved in 10 hours.

Germicidal efficacy and the time to achieve disinfection or sterilization are generally important characteristics of germicidal compositions. There is a general need in the arts for new and improved germicidal compositions containing phthalaldehyde that have higher germicidal efficacies and more rapid germicidal activity than compositions containing phthalaldehyde as the sole active ingredient.

### BRIEF DESCRIPTION OF THE SEVERAL VIEWS OF THE DRAWINGS

The invention may best be understood by referring to the following description and accompanying drawings that are used to illustrate embodiments of the invention. In the drawings;

**Figure 1** is a plot of the distribution of carbonate species, namely carbonic acid (H₂CO₃), bicarbonate (HCO₃), and carbonate (CO₃²⁻), in an aqueous solution as a function of the solution pH.

**Figure 2A** shows a container having a carbonated phthalaldehyde germicidal solution and carbon dioxide gas sealed therein, according to one embodiment of the invention.

**Figure 2B** shows an alternate container having a carbonated phthalaldehyde germicidal solution and carbon dioxide gas sealed therein, according to an alternate embodiment of the invention.

**Figure 3** shows a nano-sized or micron-sized particle containing phthalaldehyde and at least one water-soluble salt, according to one embodiment of the invention.

**Figure 4** shows a solid composition useful for preparing a germicidal solution sealed in a water-resistant container, according to one embodiment of the invention.

**Figure 5** shows an exemplary germicidal kit for preparing a germicidal solution, according to one embodiment of the invention.

**Figure 6** shows a germicidal kit to prepare a germicidal solution containing phthalaldehyde, an enhancer for the phthalaldehyde, and/or other chemicals, according to one embodiment of the invention.

**Figure 7** shows an exemplary germicidal kit including a container having a first compartment containing a solvent, a second compartment containing a solid phthalaldehyde-containing composition, and a third compartment containing an enhancer or other chemical employed with phthalaldehyde, according to one embodiment of the invention.

**Figure 8** shows a germicidal solution preparation apparatus, according to one embodiment of the invention.

### DETAILED DESCRIPTION

Described herein are germicidal compositions, kits and methods for preparing the germicidal compositions, and methods of using the compositions for disinfection or sterilization. In the following description, numerous specific details are set forth. However, it is understood that embodiments of the invention may be practiced without these specific details. In other instances, well-known structures and techniques have not been shown in detail in order not to obscure the understanding of this description.

### I. PHTHALALDEHYDE

The germicidal compositions disclosed herein include phthalaldehyde as an active ingredient. Phthalaldehyde is also known as o-phthalaldehyde, or 1,2-benzenedicarboxaldehyde, and is an aromatic dialdehyde having the structure:

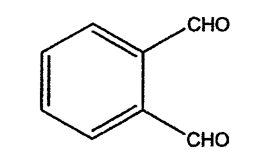

Phthalaldehyde may be used in the composition at an in-use concentration of from 0.025% to 2.0%, or 0.1 to 1% by weight. Higher concentrations, for example, up to 5% may be used if desired. Higher concentrations of phthalaldehyde may be used for shipping the composition to the point of use, and then composition may be diluted with water to the desired use concentration. The solubility of phthalaldehyde in water is about 5% by weight, which may be increased by including a water miscible, or at least more water-soluble, co-solvent. Suitable solvents include methanol, ethanol, isopropanol, n-butanol, *t*-butanol, glycols, tetrahydrofuran, dimethylsulfoxide and dioxane, among others.

The compositions may also include one or more enhancers that enhance the germicidal efficacy of the phthalaldehyde. As discussed in the following sections, the inventors have discovered that halide salts (e.g., alkali metal halide salts and polyalkylammonium halide salts), carbonates, and phosphates enhance the germicidal efficacy of phthalaldehyde.

### II. ENHANCEMENT OF THE GERMICIDAL EFFICACY OF PHTHALALDEHYDE WITH HALIDE SALTS

The inventors have discovered that halide salts enhance the germicidal efficacy of phthalaldehyde (see e.g., Examples 3-7). Based on this discovery, the inventors have developed improved germicidal compositions with greater efficacy than compositions containing phthalaldehyde alone.

In one embodiment of the invention, a germicidal composition, such as a disinfectant composition or a sterilant composition, may include phthalaldehyde and an efficacy enhancing halide salt to enhance the germicidal efficacy of the phthalaldehyde. Suitable efficacy enhancing halide salts include, but are not limited to, inorganic metal halide salts, such as alkali metal halide salts. Exemplary alkali metal halide salts include lithium halides, sodium halides, potassium halides, and combinations thereof. The halides may include fluorides, chlorides, bromides, or iodides. The inventors believe that it is the halide ions of the salts that are responsible for enhancing the germicidal efficacy of phthalaldehyde. A wide variety of exemplary halide salts are disclosed below, although the invention is not limited to these particular halide salts, and other salts or chemicals capable of liberating halide ions may also optionally be employed.

Experiments by the inventors indicate that sodium halides enhance the germicidal efficacy of phthalaldehyde. As shown in Example 4, sodium fluoride (NaF), sodium chloride (NaCl), sodium bromide (NaBr), and sodium iodide (NaI) each enhance the germicidal efficacy of phthalaldehyde. The log reductions achieved from mixtures of phthalaldehyde with the sodium halides were significantly and unexpectedly greater than the sum of the log reductions achieved when phthalaldehyde and the sodium halides were employed individually. When employed alone, a 0.3% (w/v) phthalaldehyde solution was able to achieve a log reduction of about 2.9 for *Bacillus subtilis* spores within 24 hours. The sodium halides, by themselves, had very limited, if any, germicidal activity. The sodium halides were generally able to achieve a log reduction of only about 0.2 log reduction on a 6-log scale within 24 hours. However, the log reductions for the mixtures of phthalaldehyde with the sodium halides were generally significantly and unexpectedly greater than the sum of the log reductions that were achieved when phthalaldehyde and the sodium halides are employed individually.

To illustrate, a solution including at least 0.3% phthalaldehyde and 1000 mM or more of NaF is effective at achieving a total kill of more than 6-logs of spores in only 4 hours. Further, solutions containing the same concentration of phthalaldehyde and 1000 mM or more, of either NaBr or NaI, are effective at achieving a total kill in only 8 hours. Still further, the corresponding solution containing the same concentration of phthalaldehyde and 1000 mM or more of NaCl is effective at achieving a total kill of the spores in 24 hours.

Such significant increases in the log reductions and improvement in the germicidal efficacy clearly indicates that the sodium halides enhance the germicidal efficacy of the phthalaldehyde. The enhancement may be due to a synergy or combined action on the part of the phthalaldehyde and the enhancer, such that the combined efficacy of the mixture is greater than the sum of the individual efficacies of phthalaldehyde and the halide salt enhancer. The enhancement is unexpected and significant.

Referring again to Example 4, the results seem to indicate that NaF may enhance the germicidal efficacy more than the other sodium halides, and that NaBr and NaI may enhance the efficacy more than NaCl. In one aspect, the halide salt may include a fluoride salt, such as an alkali metal fluoride salt. For example, the alkali metal fluoride salt may include lithium fluoride, sodium fluoride, potassium fluoride, or combinations thereof.

Other experiments by the inventors demonstrate enhancement of the germicidal efficacy of phthalaldehyde by other alkali metal halides. As shown in Example 5, lithium fluoride (LiF) and potassium fluoride (KF) also enhance the germicidal efficacy of phthalaldehyde. A 0.3% phthalaldehyde solution containing 1000 mM KF was effective at achieving a log reduction of 5.8 within only 4 hours, and was effective at killing more than 6-logs in 24 hours. Likewise, a 1000 mM LiF solution having the same phthalaldehyde concentration is effective at killing more than 6-logs of the spores within 24 hours.

Other suitable halide salts include, but are not limited to, alkali metal chlorides, bromides, iodides, and combinations thereof. Exemplary alkali metal chlorides include lithium chloride, sodium chloride, potassium chloride, and combinations thereof. Exemplary alkali metal bromides include lithium bromide, sodium bromide, potassium bromide, and combinations thereof. Exemplary alkali metal iodides include lithium iodide, sodium iodide, potassium iodide, and combinations thereof.

Other inorganic and organic halide salts and other materials that are capable of liberating halide ions may also optionally be employed to enhance the germicidal efficacy of phthalaldehyde. Without wishing to be bound by theory, it is believed that the halide ion component of the alkali metal halide salts plays a significant role in the enhancement, and that other materials capable of liberating halide ions will also have efficacy enhancing capabilities. It is noted that the inventors have focused largely on alkali metal halide salts due to their generally good solubility, availability, and generally low cost, although the invention is not so limited.

The inventors have performed additional experiments to determine the effect of the concentration of the halide salt on the enhancement of germicidal efficacy. Example 3 shows that a higher halide salt concentration, at least in the case of sodium fluoride (NaF), generally provides greater enhancement over the range from 100 to 1000 mM. It was found that a solution including at least 0.3% phthalaldehyde and 1000 mM or more of NaF is effective at achieving a total kill within 4 hours, whereas a solution including 400 mM or more of NaF is effective at achieving a total kill within 8 hours, and a solution including 100 mM or more of NaF is effective at achieving a total kill of the spores within 24 hours.

In general, the inventors contemplate employing halide salt enhancers at various concentrations sufficient to achieve a desired degree of enhancement. Typically, the in-use concentration of the halide salt enhancer is from at least about 100 mM to a saturated concentration. It is difficult to place a definite circumference on the saturation concentration of all suitable salts, since this may depend on the solubility of the particular salt, the temperature, and the presence or absence of other species, among other factors. However, saturation concentrations may easily be determined by measurement, by those skilled in the art, without undue experimentation. In one aspect, the halide salt may be employed at an in-use concentration of from at least 500 mM to 1000 mM, or higher (e.g., 2000 mM). A higher concentration of the halide salt generally provides greater enhancement.

For relatively low solubility chemicals, such as certain organic and inorganic halide salts, the amount of enhancement may be somewhat limited by the solubility or concentration of the halide ions. If desired, a solubility enhancer may be employed to enhance the solubility or concentration of at least the halide ions. For example, EDTA or another complexing or chelating agent may be added to complex the cation of the halide salt, and thereby shift the equilibrium in favor of an increased concentration of the halide ions. As another option, a plurality of different halide salts may be employed to provide an increased combined concentration of halide ions. For example, a combination of calcium chloride (CaCl₂), magnesium fluoride (MgF₂), aluminum fluoride (AlF₃), tetrabutylammonium fluoride [CH₃(CH₂)₃]₄NF and tetrabutylammonium chloride [CH₃(CH₂)₃]₄NCl may be employed together to increase the total concentration of halide ions. Such approaches may help to provide higher concentrations of the halide ions, and generally provide greater enhancement.

It may be helpful to review, that as previously discussed, the phthalaldehyde may be employed at a germicidally effective concentration. Typically, an in-use concentration of the phthalaldehyde is from at least about 0.025% (w/v) to about a saturation concentration. Often, the in-use concentration of the phthalaldehyde is from about 0.1 to 1% (w/v).

The inventors have performed additional experiments to determine the effect of pH or alkalinity on the enhancement of germicidal efficacy. Experiments indicate that the enhancement of germicidal efficacy may increase with increasing pH or alkalinity. As shown in Example 6, a higher pH generally enhances the germicidal efficacy of a phthalaldehyde solution including an alkali metal halide salt, at least in the case of potassium fluoride (KF), over the pH range from 6.6 to 10.1. At a pH of 10.1, the solution was able to achieve a total kill of more than 6-logs of spores in only 4 hours.

To achieve good disinfection or sterilization, it may be appropriate to provide an in-use pH of from about 6 to 10. Often it may be appropriate to provide a composition having an in-use pH that is at least 6.5, at least 7, at least 7.5, or at least 8, in order to achieve greater germicidal efficacies. Even higher pH up to about 11 may be employed, although such high or alkaline pH may potentially damage certain materials, such as rubber, during disinfection or sterilization. In certain cases, depending upon the application, it may be appropriate to maintain an in-use pH that is less than 9, or more often less than 10, to provide greater compatibility with rubber and other materials.

Acids, bases, buffers or other pH adjusters may optionally be employed for any desired pH adjustment. The pH adjuster employed in Example 6 was a base, namely sodium hydroxide (NaOH), or an acid, such as hydrochloric acid, although other pH adjusters may also optionally be employed. Other examples of suitable pH adjusters or buffers that may be employed in the germicidal composition include, but are not limited to, borax plus HCI, carbonate plus hydrogen carbonate, diethylbarbiturate (veronal) and HCl, KH₂PO₄ plus borax, N-2-hydoxythylpiperazine-N'-2-ethanesulfonic acid and NaOH, and phosphate. Still another exemplary pH adjuster is a phosphate buffer, such as the KH₂PO₄ and Na₂HPO₄ phosphate buffer, which is able to buffer a pH in a range from about 6 to 7.5. Another exemplary pH adjuster is EDTA (ethylenediaminetetraacetic acid) in a free acid, mono-, di-, tri-, or tetra-salt form, or a buffer including a combination of such forms, which allow buffering over a pH range from about 3 to 10. The EDTA may also serve as a chelating agent to help prevent precipitation. For example, other alkalinating or acidifying agents, such as organic carboxylate salts (e.g., sodium citrate, sodium acetate, potassium hydrogen phthalate, potassium citrate, potassium acetate), inorganic borate salts (e.g., potassium borate or sodium borate), and mixtures of such agents, may potentially be employed. It will be appreciated that such buffers may also optionally be employed in the other compositions disclosed herein. The pH adjusters may be present in a sufficient amount, for example 0.05wt% to 2.5wt%, to give a desired pH.

The inventors have discovered that certain combinations of halide and other salts provide even greater enhancement of the germicidal efficacy of phthalaldehyde. As shown in Example 7, certain sodium halide salts, such as sodium chloride (NaCl), sodium bromide (NaBr), and sodium iodide (NaI), and other sodium salts, such as sodium sulfate (Na₂SO₄), may enhance the germicidal efficacy of a solution including phthalaldehyde and sodium fluoride (NaF). The log reductions for the mixed composition of phthalaldehyde, NaF, and these salts, namely 5.6, 5.9, 5.9, and >6.0, are each significantly greater than the log reduction of 4.7 observed when the salts NaCl, NaBr, NaI, and Na₂SO₄, respectively, were omitted from the composition. If desired, a halide salt enhancer and one of the salts NaCl, NaBr, NaI, or Na₂SO₄ may be employed in combination or concert in a germicidal solution with phthalaldehyde in order to provide further enhancement.

The inventors have performed experiments to determine the material compatibility of germicidal compositions including various halide salt enhancers to common materials. As shown in Example 8, alkali metal halides, such as sodium halides and potassium halides, are compatible with stainless steel and DuPont™ Teflon® brand polytetrafluoroethylene over a period of 72 hours, as determined by visual inspection. Stainless steel and Teflon® are widely used materials in the medical devices and other industries. In one aspect, the results demonstrate that the disclosed compositions may be used to disinfect or sterilize a surface or device including stainless steel, or Teflon®. For example, the disclosed compositions may be used to disinfect or sterilize an endoscope containing stainless steel or Teflon®.

Specific examples of germicidal compositions including phthalaldehyde and halide salt enhancers are disclosed in Examples 18-22. Each of the compositions is able to achieve a total kill of all tested *Bacillus subtilis* spores within only 4 hours.

If desired, the phthalaldehyde plus halide salt enhancer composition may additionally contain one or more other enhancers disclosed herein. For example, the composition may include bicarbonate, or carbonate. If desired, the composition may be provided as a carbonated or solid composition to help maintain stability of the phthalaldehyde during storage, as will be further explained below. The use compositions may also be prepared from a kit, such as those disclosed below, in which the phthalaldehyde is employed as a first composition, either a solid composition or a liquid composition, and the halide salt enhancer is employed as a discrete second composition. The compositions may be included in separate containers or compartments. In the case of a solid composition, the kit may optionally contain a solvent, for example, separated in a container or compartment, to help dissolve the solid composition.

### III. ENHANCEMENT OF THE GERMICIDAL EFFICACY OF PHTHALALDEHYDE WITH CARBONATES

The inventors have discovered that carbonates, such as carbonate salts and bicarbonate salts, enhance the germicidal efficacy of phthalaldehyde (see Examples 9-17). Based on this discovery, the inventors have developed improved germicidal compositions with greater efficacy than compositions containing phthalaldehyde without an enhancer.

In one embodiment of the invention, a germicidal composition, such as a disinfectant composition or a sterilant composition, may include an aqueous solution containing phthalaldehyde and a carbonate enhancer. Suitable carbonate enhancers include, but are not limited to, carbonate salts, bicarbonate salts, and combinations thereof.

Suitable carbonate salts include, but are not limited to, sodium carbonate (Na₂CO₃), potassium carbonate (K₂CO₃), calcium carbonate (CaCO₃), magnesium carbonate (MgCO₃), lithium carbonate (Li₂CO₃), and combinations thereof. Suitable bicarbonate salts include, but are not limited to, sodium bicarbonate (NaHCO3), potassium bicarbonate (KHCO₃), lithium bicarbonate (LiHCO₃), and combinations thereof. Species such as carbon dioxide (CO₂) and carbonic acid (H₂CO₃) are also suitable sources of a carbonate enhancer, as will be discussed further below.

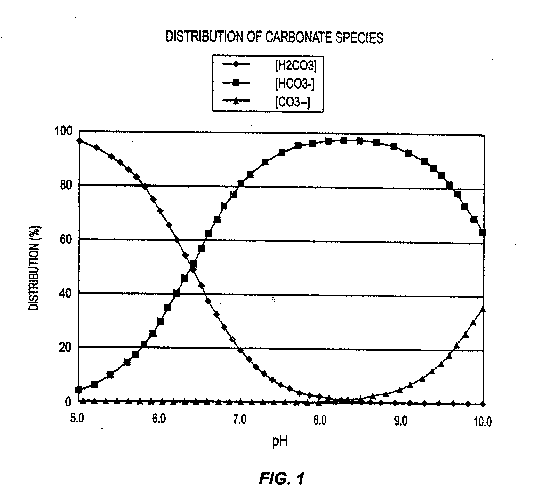

**Figure 1** is a plot of the well-known equilibrium distributions of carbonate species, namely carbonic acid (H₂CO₃), bicarbonate (HCO₃), and carbonate (CO₃²⁻), in an aqueous solution as a function of the solution pH. The distribution of the species is plotted on the y-axis and the solution pH is plotted on the x-axis. The carbonate species exist in equilibrium in the solution at concentrations that depend upon the solution pH. Carbonic acid predominates at pH less than about 6.4, whereas bicarbonate predominates at pH greater than about 6.4. At pH greater than about 8.3, the concentration of carbonate begins to steadily increase. As one example of reading the plot, at a pH of about 7.0, the distribution of carbonate species in an aqueous solution is about 80% bicarbonate, 20% carbonic acid, and less than 1% carbonate.

The plot shows several conversions that may be employed in aspects of the invention, as will be further discussed below. The introduction of carbon dioxide into solution may form carbonic acid by hydration. In one aspect, the carbonic acid may be converted into bicarbonate and carbonate by raising the solution pH. In another aspect, bicarbonate or carbonate solution may be carbonated, by converting a portion of the bicarbonate or carbonate to carbonic acid, by lowering the pH. The carbonated solution may be sealed in a pressurized container to retain the carbonation.

Experiments by the inventors indicate that carbonate and bicarbonate each enhance the germicidal efficacy of phthalaldehyde. As shown in Example 9, the killing of the spores, as evidenced by the log reductions, is enhanced by carbonate and bicarbonate. The enhancement increases with increasing bicarbonate concentration. A concentration of sodium bicarbonate of 63 mM or higher is sufficient to achieve sterilization as represented by a total kill of all spores within 24 hours. The enhancement is unexpected and significant. Overall, the inventors observed negligible log reductions when carbonates or bicarbonates were employed without phthalaldehyde. The log reductions for the mixtures of phthalaldehyde with the carbonates or bicarbonates were generally significantly and unexpectedly greater than the sum of the log reductions that were achieved when phthalaldehyde and the carbonates are employed individually. The enhancement is significant and unexpected.

To put the current research noted above in context (and to help the reader in understanding the significance of the present discovery), it may be helpful to briefly recite several investigations that led to today's understanding of the effect of carbonates and bicarbonates. Two recent investigations reported in the literature demonstrate that carbonates apparently do not enhance the efficacy of some aldehydes, such as formaldehyde or butyraldehyde, while they apparently do enhance the efficacy of others, such as glutaraldehyde. This seems to indicate that there is a high level of unpredictability of the affect of carbonates on the efficacy of various aldehyde-based germicides.

E.G.M. Power and A.D. Russell, in the article entitled, "*Sporicidal Action of Alkaline Glutaraldehyde: Factors Influencing Activity and Comparison With Other Aldehydes*" (Journal of Applied Bacteriology, 69, pp. 261-268, 1989) investigated in part the sporicidal action of 2% alkaline glutaraldehyde at room temperature and the sporicidal action of other aldehydes, such as formaldehyde, glyoxal, and butyrladehyde, and commercially available formulations. They reported in part that the increased sporicidal efficacy of alkaline glutaraldehyde is due to more than a simple pH effect, and that the addition of NaOH to acid glutaraldehyde does not increase biocidal activity to the same extent as does the addition of NaHCO₃. They also reported that the addition of 0.3% (w/v) NaHCO₃ to glyoxal and butyraldehyde did not affect their sporicidal action. Phthalaldehyde was not investigated.

Jose-Luis Sagripanti and Aulin Bonifacino, in the article entitled, "*Effects of Salt and Serum on the Sporicidal Activity of Liquid Disinfectants*" (Journal of AOAC International, 10(6), pp. 1198-1207, 1997) reported in part the effects of various concentrations of salt or serum in the killing of *Bacillus subtilis* spores by either glutaraldehyde, sodium hypochlorite, cupric ascorbate, hydrogen peroxide, peracetic acid, formaldehyde, or phenol. Salt affected only glutaraldehyde, its sporicidal activity increasing with an increase in concentration of sodium bicarbonate or sodium chloride. Sporicidal activities of peracetic acid, sodium hypochlorite, hydrogen peroxide, and cupric ascorbate, as well as the low sporicidal activities of phenol and formaldehyde, were not affected by variations in salt ranging from 0 to 1M. Accordingly, bicarbonate and sodium chloride affects some but not all disinfectants, including some but not all aldehydes. Phthalaldehyde was not included in the investigation.

Referring again to the experiments of the inventors, and in particular to Example 9, the carbonate and bicarbonate salts were sodium and potassium salts, respectively. Other experiments by the inventors demonstrate enhancement of the germicidal efficacy of phthalaldehyde by other alkali metal carbonates and bicarbonates. As shown in Example 11, other alkali metal carbonates, such as lithium carbonate, are also suitable enhancers. Three different solutions are listed which achieved a total kill of the spores in only 4 hours.

Still other experiments by the inventors demonstrate that species such as carbon dioxide (CO₂) and carbonic acid (H₂CO₃) are also suitable sources of carbonate enhancer. As shown in Example 12, purging carbon dioxide through an alkaline solution provides a suitable carbonate for enhancing the germicidal efficacy of phthalaldehyde. Other species capable of being reacted to produce carbon dioxide, carbonic acid, carbonate, or bicarbonate are also potentially suitable.

Referring again to Example 9, among others, the enhancement increases with increasing bicarbonate or carbonate concentration. Typically, the in-use concentration of the carbonate enhancer is from about 10 mM to a saturated concentration. The saturation concentrations may readily be determined by measurement, by those skilled in the art, without undue experimentation. In one aspect, the in-use concentration of the carbonate or bicarbonate enhancer is from about 50 mM to 500 mM. Experiments conducted at the same pH indicate that higher carbonate concentrations generally give greater enhancement.

The inventors have performed additional experiments to determine the effect of pH or alkalinity on the enhancement of germicidal efficacy.. Experiments indicate that the enhancement of germicidal efficacy may increase with increasing pH or alkalinity. As shown in Example 10, a higher or more alkaline pH, at least over the range from 8.2. to 10.3, generally enhances the killing of spores by a solution containing phthalaldehyde and bicarbonate.

To achieve good disinfection or sterilization, it may be appropriate to provide an in-use pH of from about 6 to 10. Often it may be appropriate to provide a composition having an in-use pH that is at least 6.5, at least 7, at least 7.5, or at least 8, in order to achieve greater germicidal efficacies. Even higher pH up to about 11 may be employed, although such high or alkaline pH may potentially damage certain materials, such as rubber, during disinfection or sterilization. In certain cases, depending upon the application, it may be appropriate to maintain an in-use pH that is less than 9, or more often less than 10, to provide greater compatibility with rubber and other materials. In one aspect, the in-use pH may be from about 7.5 to 9 to provide good enhancement and material compatibility. Acids, bases, buffers, or other pH adjusters may be employed for any desired pH adjustment. The pH adjusters may be present in a sufficient amount, for example 0.05wt% to 2.5wt%, to give a desired pH.

The inventors have determined a number of additional salts that are efficacy enhancers for phthalaldehyde, or mixtures of phthalaldehyde and carbonate. As shown in Example 13, phosphate enhances the killing of spores by phthalaldehyde when employed with bicarbonate. The phosphate appears to provide a very slight enhancement without bicarbonate..

A variety of halide salts apparently also enhance the killing of spores by phthalaldehyde when employed with bicarbonate. As one example, as shown by Example 14, the potassium halides, namely potassium chloride (KCl), potassium bromide (KBr), potassium iodide (KI), or potassium fluoride (KF), enhance the killing of spores by phthalaldehyde when employed with bicarbonate.

Other alkali metal halides, such sodium halides, also enhance the killing of spores by phthalaldehyde. As shown by Example 15, sodium halides may enhance the germicidal efficacy of phthalaldehyde when employed with or without bicarbonate. Even at low concentrations, several sodium halides, namely sodium fluoride (NaF), sodium bromide (NaBr), and sodium iodide (NaI), may enhance the killing of spores by phthalaldehyde, when employed without bicarbonate. Also, at the same low concentrations, several of the sodium halides, namely sodium chloride. (NaCl) and sodium fluoride (NaF), may enhance the killing of spores by phthalaldehyde, when employed with bicarbonate.

The enhancement provided by sodium chloride (NaCl) was further investigated in Example 16. The sodium chloride (NaCl) enhanced the killing of spores by phthalaldehyde when employed with bicarbonate. The enhancement begins to become noticeable at a concentration of from 50 to 100 mM, and the enhancement increases with concentration to at least 200 mM.

Still further, as shown in Example 17, polyalkylammonium halides such as n-tetrabutylammonium fluoride (Bu₄NF), n-tetrabutylammonium chloride (Bu₄NCl), n-tetrabutylammonium bromide (Bu₄NBr), and *n*-tetrabutylammonium iodide (Bu₄NI) enhance the killing of microorganisms with phthalaldehyde when employed with bicarbonate. Bu₄NCl, and Bu₄NBr appear to provide slightly greater enhancement than Bu₄NF and Bu₄NI under the conditions tested.

In one aspect, one or more of these enhancers, namely phosphate, alkali metal halides, and polyalkylammonium halides, may be included in a phthalaldehyde plus carbonate or bicarbonate germicidal composition to enhance the germicidal efficacy of phthalaldehyde and improve disinfection or sterilization. As one example, phosphate and sodium bicarbonate may be included in a composition with phthalaldehyde to enhance the efficacy of the phthalaldehyde. A potential advantage of these enhancers is an ability to reduce the bicarbonate or carbonate concentration. Among other motivations, carbonate reduction may help to simplify manufacturing and packaging requirements, due in part to reducing potential for carbon dioxide evolution, and help to avoid insoluble calcium and magnesium carbonate salts with hard water.

### IV. KILLING MICROORGANISIMS, DISINFECTION, AND STERILIZATION

The germicidal compositions may be used as either disinfectants or sterilants. A disinfectant generally refers to a material capable of killing all non-spore microbes but not spores. High-level disinfectant generally refers to a material capable of killing some spores, such as *Bacillus subtilis* and *Clostridium sporogenes,* in addition to killing non-spore microbes. A sterilant generally refers to a material capable of killing all spores and non-spores.

A method of using the composition for disinfection or sterilization may include contacting microorganisms with the composition, or otherwise applying the composition to the microorganisms, either in the air, on surfaces, or in other fluids, in order to kill the microorganisms. The composition may be applied to the air by spraying, applied to a surface by immersion, spraying, coating, flowing, or the like, or applied to a fluid by combining the composition with the fluid, for example. Often, the composition may be employed to disinfect or sterilize a surface by contacting the surface with the composition, such as by immersion, spraying, coating, or flowing the composition over the surface for a period of time and at a temperature effective to achieve disinfection or sterilization of the surface. The composition may be employed manually, for example in a processing basin, or by an automated system, such as an automated endoscope reprocessor (AER). Generally, the solutions have the advantages of allowing disinfection or sterilization without expensive capital sterilization equipment, are easy for health personnel to use, and are effective and reliable.

The degree of effectiveness of germicides is typically influenced by the in-use concentrations of active ingredients, treatment time, temperature, and test method. U.S. Pat. No. 4,971,999, issued November 20, 1990, to Bruckner et al., discloses in part that compositions that contain at least 0.25% by weight phthalaldehyde as the sole active ingredient are effective to achieve high-level disinfection as determined by the ability of said composition to kill all *Mycobacterium bovis* BCG in contact with the composition within 10 minutes at 20°C. At about the same phthalaldehyde concentration and temperature, the compositions disclosed herein, which also include one or more enhancers for phthalaldehyde, may achieve high-level disinfection in an even shorter period of time.

The '999 patent also discloses that compositions containing a low concentration of phthalaldehyde (e.g., 0.25%) as the sole active ingredient has sporicidal activity against *Bacillus subtilis* and *Clostridium sporogenes* spores in 24 hours at a temperature of 20°C. At higher concentrations (e.g., 1.0%) of phthalaldehyde, sterilization is achieved in 10 hours. The sterilization results presented in the '999 patent are based on the AOAC (Association of Official Analytical Chemists) Sporicidal Test, as specified in Official Methods of Analysis of the Association of Official Analytical Chemists, 14th Edition, 1984. See e.g., Examples 8 and 9 in the '999 patent.

Some researchers believe that the AOAC test may not be sufficiently quantitative and may lead to highly erratic and variable times to achieve disinfection or sterilization. A potential problem cited by these researchers is that the number of spores on the carrier may be highly variable. For example, Danielson (Evaluation of Microbial Loads *of Bacillus Subtilis* Spores on Penicylinders, J. AOAC Int, 76:355-360, 1993) has reported that a carrier may contain as few as only 500 spores, or about 2.7-logs, and meet the AOAC criteria. It is generally accepted that the performance of a sporicide may depend on the number of spores to be killed. This would mean that a small number of spores, such as only 500 spores, may be killed much more quickly than a large number of spores, say at least 1,000,000 spores (at least 6-logs).

The experiments performed herein, unless specified otherwise, are based on 6-logs of spores, and should provide more accurate, and more quantitative estimates of the time to achieve disinfection or sterilization. This means that it may be difficult to directly compare the times for disinfection or sterilization reported in the '999 patent, which are based on the AOAC test, with the times reported herein, which are based on the improved suspension test. However, in any event, at about the same phthalaldehyde concentration and temperature, the enhanced compositions disclosed herein may achieve disinfection or sterilization more effectively and rapidly than the compositions disclosed in the '999 patent, using the same test.

### V. CHEMICAL STABILITY OF PHTHALALDEHYDE

Storage stability and ease of product use are two important considerations when selecting sterilizing and high level disinfecting solutions. As discussed in U.S. Pat. No. 3,016,328, and in U.S. Pat. No. 4,971,999, glutaraldehyde and other similar aldehydes with α-hydrogens may autopolymerize at an alkaline pH. Compositions containing these aldehydes at an alkaline pH may experience a reduction in the effective concentration of the aldehyde with time and, therefore, may have limited storage stability. In order to overcome this problem, the glutaraldehyde compositions have been packaged in two or more components. The aldehydes may be formulated in an aqueous solution at an acidic pH, and activated with an alkalinating agent immediately prior to use, shifting the pH to the alkaline range.

As further discussed in the '999 patent, unlike the aforementioned aldehydes, phthalaldehyde does not have α-hydrogens, and therefore generally does not undergo autopolymerization at an alkaline pH. Still further, it is discussed in the '999 patent that the compositions containing phthalaldehyde are generally formulated as a single component, and have excellent stability over a pH range of 3 to 9. They do not lose their effectiveness during storage.

However, the inventors have realized that alkaline phthalaldehyde solutions may be relatively chemically unstable over prolonged periods of storage, especially under more alkaline conditions, due to the tendency of phthalaldehyde to participate in the well-known Cannizzaro reaction.

Overall, the Cannizzaro reaction generally leads to loss of phthalaldehyde and a decrease in the germicidal efficacy of the solution. While a pH of from 6 to 10, or 7.5 to 9 generally enhances the efficacy of the phthalaldehyde-carbonate solution, the higher or alkaline pH also generally promotes the Cannizzaro reaction. Experiments indicate that a phthalaldehyde solution may be stored for about 11 weeks at a pH of 7 or lower, and at room temperature to about 40°C, without a noticeable loss of phthalaldehyde. However, about 14% of the phthalaldehyde may be lost if the same solution is stored for about 11 weeks at a pH of 9, at room temperature. Even more of the phthalaldehyde may be converted if the storage period is longer, if the temperature is higher, or if the pH is higher than 9. Thus, the Cannizzaro reaction may significantly decrease the efficacy or shelf life of an alkaline phthalaldehyde solution during typical periods of storage used in the arts.

The inventors have developed several approaches, which are disclosed in the following sections, to allow phthalaldehyde to be stored for prolonged periods without significant loss of efficacy, and then employed as a germicidal solution having an alkaline pH that enhances the germicidal efficacy.

### VI. CARBONATED GERMICIDAL SOLUTIONS

According to another embodiment of the invention, a carbonated germicidal solution containing phthalaldehyde may be sealed in a container. The inventors have discovered that carbonation may help to improve the chemical stability of phthalaldehyde. When carbonated, or charged with CO₂, the germicidal solution may have an acidic pH, such as a pH that is less than about 6, which promotes chemical stability of the phthalaldehyde by helping to suppress the Cannizzaro reaction. Then, when needed, the sealed container may be opened, allowing the solution to become de-carbonated. The de-carbonation of the solution may automatically increase the pH of the solution, for example to a pH from about 6 to 10, or 7.5 to 9. Such a high or alkaline pH may enhance the germicidal efficacy of the phthalaldehyde.

Carbonation generally involves introducing or impregnating carbon dioxide into a solution. Carbon dioxide is a plentiful and relatively cost effective gas that is commercially available from numerous sources, including but not limited to Praxair, Inc of Danbury, Connecticut. An exemplary method of making a pressurized germicidal solution in a sealed container, according to one embodiment of the invention, may include combining phthalaldehyde and any other optional ingredients (for example an enhancer) with the solution, introducing the carbon dioxide gas into the solution, introducing the solution into the container, and then sealing the container. The phthalaldehyde and carbon dioxide may be introduced into the solution in any desired order, and this may be performed before, after, or during introduction of the solution into the container. Various approaches for introducing carbon dioxide into liquids, including water, are known in the arts. In the carbonated water industry, approaches such as bubbling, sparging, agitation, or mixing are often used to improve contact between the carbon dioxide and the water. Such approaches may be used to introduce the carbon dioxide into the germicidal solution. A solid form of carbon dioxide, such as dry ice, may also be introduced into the solution to introduce carbon dioxide into the solution.

Another method of introducing or impregnating carbon dioxide into the solution may include combining a carbonate or bicarbonate salt with the solution. The carbonate or bicarbonate salt may be introduced into an acidic solution, or may be introduced into the solution with an acidifying agent, to cause the salt to react to produce carbonic acid and carbon dioxide in *situ* in the solution. Such a method may avoid the need to handle gaseous carbon dioxide. A specific example of a carbonated germicidal solution that may be produced by such a method is shown in Example 23.

Once introduced, the carbon dioxide may help to acidify the solution. In an aqueous solution, the introduced carbon dioxide may react with water to form carbonic acid. Enough carbon dioxide may be introduced to achieve a pH that helps to suppress the Cannizzaro reaction during storage. In an acidic solution, the Cannizzaro reaction proceeds relatively slowly, and the stability of such acidic solutions is significantly better than the stability of a neutral or alkaline solution. In one aspect, enough carbon dioxide may be introduced to reduce the pH to less than about 8 or 6. In another aspect, the solution may be substantially saturated with carbon dioxide. If desired, the solution may optionally be cooled and pressurized to increase the solubility of the carbon dioxide. The carbonated solution in the container may be distributed to a point of use, and there stored until needed. Such carbonated solutions should be substantially more stable than alkaline solutions, and may be stored for longer periods of time.

Referring again to the distribution of carbonate species in an aqueous solution, which is shown in Figure 1. It is seen that the carbonate species exist in equilibrium in the solution at concentrations that depend upon the solution pH. The introduction of carbon dioxide into solution may form carbonic acid, which tends to lower the solution pH. The plot also shows that carbonic acid may be converted to bicarbonate or carbonate by raising the pH.

When needed, a user may obtain the container from storage. A method, according to one embodiment of the invention, may include opening the container, removing the carbonated solution from the container, and disinfecting or sterilizing a surface by contacting the surface with the carbonated solution. As with carbonated beverages, soon after opening the container, bubbles of carbon dioxide may begin to form and evolve from the solution due to favoring the conversion of carbonic acid back into dissolved carbon dioxide at ambient pressure. The bubbles may potentially help to enhance disinfection or sterilization by lifting or otherwise carrying contaminants, such as dirt, microorganisms, or spores, away from the surface or device being treated.

Generally coincident with the formation of the bubbles, the pH of the solution may begin to increase, and may become alkaline, as carbonate and bicarbonate are formed. The amount of carbonation, carbonate or bicarbonate, and any other pH adjusters may be balanced to achieve a de-carbonated pH of from about 6 to 10, or about 7.5 to 9. As discussed above, such a high or alkaline pH may enhance the efficacy of the phthalaldehyde, and lead to improved disinfection, or sterilization. Even higher pH up to about 11 may be achieved, by including more carbonate or a similar alkalinating agent in the germicidal solution, although such high pH are generally avoided due to potential corrosion of materials during disinfection or sterilization.

With reference to **Figure 1**, when performing disinfection or sterilization at a pH that is less than about 8, and especially less than about 6.5, the pH may tend to increase, and carbonate enhancer may be lost, due to the ability of carbonic acid to form carbon dioxide, which may tend to evolve and escape. If desired, above-ambient pressure may be provided, such as in a pressurized chamber, to help suppress the evolution of the carbon dioxide, and stabilize the pH. This may help to retain enhancement with carbonate over prolonged periods. Alternatively, a pH adjuster, such as an EDTA buffer, may be employed to help stabilize the pH below 7.5. As yet another option, an acidifying agent or pH adjuster, such as carbon dioxide, may be added to the solution regularly, or based on pH control, to maintain the pH below about 7.5.

The chemical stability of the phthalaldehyde, and the efficacy of the solution, may be checked or confirmed by examining for pressure or bubbles during or after opening the container. Generally, when the container having the carbonated solution is opened, there should be an indication of pressure, such as a sound of gas escaping the container, and bubbles of carbon dioxide should form and evolve from the container soon after opening. The pressure and the bubbles generally indicate an appropriate amount of carbonation, a correspondingly low pH, and confirm that the container does not have a leak or other defect, which would allow carbon dioxide to escape. As discussed above, the carbonation helps to lower the pH and increase the chemical stability of the phthalaldehyde. The pressure and the bubbles generally confirm the efficacy of the solution. In contrast, the absence of pressure or bubbles may be indicative of a high or alkaline pH, and may potentially indicate that the efficacy of the solution has been compromised during storage due to the Cannizzaro reaction, or that the solution was initially insufficiently carbonated.

In one aspect, a container may have a label attached thereto that contains information associating an efficacy or quality of the solution contained therein with an indication of pressure (such as a sound of gas escaping the container as it is opened), or the occurrence of bubbles in a recently opened container, or both. The label may contain information instructing a user to discard the solution if the pressure or the bubbles are not present. For example, the label may essentially say "discard solution if no bubbles form after opening container". A user of the germicidal solution may read the label, open the container, and either examine for pressure (for example listen for the sound of gas escaping as the container is opened), or examine the solution for bubbles after opening the container, or both, as an indication of the quality or efficacy of the germicidal solution. Based on the indicated examination, the user may use the solution to disinfect or sterilize a surface, if the pressure or bubbles are confirmed, or otherwise discard the solution.

As another option, the container may include a pressure indicator to indicate whether or not the container has a pressure greater than an ambient pressure. For example, the container may have an outward half-ball shell formed on a surface thereof to allow a user to test whether the container is pressurized. Under normal storage conditions the outward half-ball shell should bias outward. The user may depress the half-ball shell inward, toward the inside of the container. The half-ball shell may either deflect back outward, if the container has an internal pressure greater than an ambient pressure, or remain depressed inward, if the container is un-pressurized, or has insufficient pressure. In one aspect, the lowest internal pressure at which the half-ball shell may deflect back outward may be based on a level of carbonation corresponding to a solution pH that provides stability for at least a predetermined minimum effective concentration (MEC) of phthalaldehyde over a predetermined or guaranteed storage period. Other pressure indicators that may also potentially be employed include, but are not limited to, pressure gauges, piezoelectric devices, and other pressure indicators known in the arts.

As yet another option, a user may measure, test, or otherwise ascertain the pH of a recently opened solution to determine if the pH is inappropriately elevated due to escape of carbon dioxide during storage. A pH meter, a pH test strip, or other pH sensitive materials may be employed. An inappropriately high pH may indicate a loss of carbonation and a potential decrease in phthalaldehyde concentration due to promotion of the Cannizzaro reaction at alkaline pH.

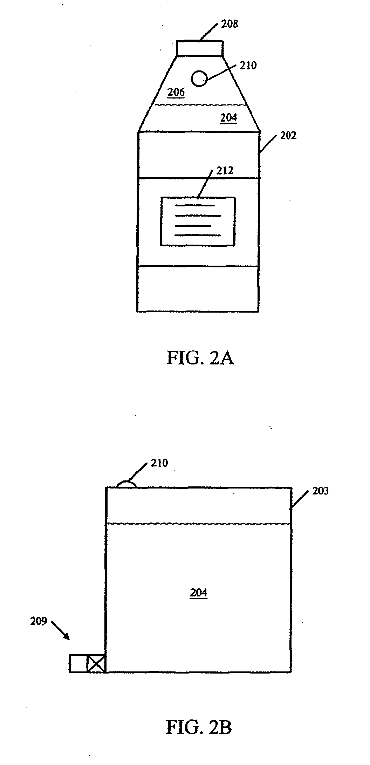

**Figure 2A** shows a container 202 having a carbonated phthalaldehyde germicidal solution 204 and carbon dioxide gas 206 sealed therein, according to one embodiment of the invention. The container may be a glass, metal (e.g., aluminum) or especially a plastic container, and includes a cap 208 that may be opened to remove the germicidal solution from the container. In one aspect, the container may include a transparent or translucent material to allow inspection of the solution in the container for bubbles. A pressure indictor 210, such as a half ball shell, is formed on the surface of the container. The container also has a label 212 that may include instructions on how to examine the solution prior to use. The container may be designed to accommodate an internal pressure of carbon dioxide gas, for example in a range between 1 to 50 psi, or 5 to 30 psi. Using the carbonate species distribution curves shown in Figure 1, the pressure of carbon dioxide may be estimated from factors such as the total amount of carbonate, pH, temperature, solubility of carbon dioxide in the solution, the volume of the solution, and the gas volume in the container.

The invention is not limited to any known size or shape of the container. **Figure 2B** shows a container 203, according to an alternate embodiment of the invention, which has a different shape, and a larger size. A valve-controlled opening 209, such as a stopcock controlled opening, may be used to remove or dispense portions of the germicidal solution 204 from the container. The large size and the stopcock may allow portions of the solution to be removed from the container as needed. Since the stopcock is located proximate the bottom of the container, there is a significant amount of liquid above the stopcock. The liquid above the stopcock may help to provide a head or pressure to help keep the container 'sealed', even after opening the container to remove some of the solution, and help to keep the solution at least partially carbonated. Thus, the unused portion of the solution in the container may retain an acidic pH, and may be used for longer periods of time, even after the container has been opened.

### VII. SOLID COMPOSITIONS

The inventors have developed solid compositions containing phthalaldehyde that may be distributed to a point of use, stored, and then used to prepare germicidal solutions that are useful for disinfection, or sterilization. The Cannizzaro reaction generally occurs slowly, if at all, in dry solids due to the absence of water, which tends to promote the reaction. Accordingly, the solid compositions provide a chemically stable environment for storing phthalaldehyde, even if the phthalaldehyde is present in the composition with generally alkaline components, such as a carbonate salt. Other potential advantages of the solid composition include reduced transportation costs and storage space due to the elimination of solvent.

According to one embodiment of the invention, a solid composition may include a solid salt, and a solid phthalaldehyde dispersed or otherwise diluted in the solid salt. The dilution of the phthalaldehyde in the salt may help to reduce clumping or other forms of aggregation of the phthalaldehyde. The favored salt may have higher water solubility than phthalaldehyde to help dissolve the solid composition in the solvent. In one aspect, the salt may include an efficacy enhancing salt for phthalaldehyde, such as a carbonate, phosphate, alkali metal halide salt, polyalkylammonium halide salt, or a combination of such salts. In another aspect, a highly water-soluble salt, whether or not it is enhancing, such as sodium sulfate (Na₂SO₄), may be employed. Soluble non-salts such as starch or cellulose may also optionally be employed.

Other optional ingredients that may be included in the solid composition include a pH adjuster, a chelating agent (e.g., EDTA), a corrosion inhibitor (e.g., benzotriazole), a surfactant, a dye, and a fragrance, among others. Suitable pH adjusters include, but are not limited to, phosphate buffers, bicarbonate buffers, carboxylic acid/salt buffers, such as EDTA buffers, HCl, and NaOH. The adjusters may be employed in amounts sufficient to adjust a pH of the germicidal solution to a pH in a range between 6 to 10, or 7.5 to 9, for example.

In the solid formulation, the Cannizzaro is so unlikely to happen that a solid with high (basic) Solid Potential pH (SPP) may be designed. The SPP is the potential pH upon dissolving the solid composition in water. The advantages include a solid composition that provides a stable storage environment for phthalaldehyde and has the potential to cause a high (basic) pH once dissolved in water to enhance the efficacy of the phthalaldehyde. Likewise, a solid acid with low (acidic) SPP, such as organic acid (for example citric acid, ascorbic acid, etc.) may be mixed with OPA to produce a low (acidic) SPP solid composition. This may provide an alternative to using a pressurized container. Solid compositions with either high SPP or low SPP may have different applications. Both may have high stability and long shelf life. This may be especially advantageous for shipment and storage at higher temperatures (e.g., without air conditioning).

Generally, the solid composition may include micron-sized or nano-sized particles or other finely divided portions of phthalaldehyde to facilitate dissolution of the phthalaldehyde. In one aspect, the particles may include nanoparticles having a size that is less than about 100 nanometers. The particles or nanoparticles may be prepared by grinding, milling, spray drying, or other approaches known in the arts (e.g., potentially using a Raleigh jet, or spinning-disk atomizer). The particles may also be formed by super-critical gas drying, such as super-critical carbon dioxide drying.

In grinding, particles of phthalaldehyde, or a phthalaldehyde powder, may be formed by breaking larger portions of solid phthalaldehyde in a grinding device. Suitable grinding devices include, but are not limited to, mortars and pestles, mechanical grinding devices, mills, ball mills, and air-jet mills. A method of preparing a powder, according to one embodiment, may include placing solid phthalaldehyde and a salt, such as bicarbonate, in a grinding device, such as a caged rotating device containing metal or ceramic balls, such as a ball mill, and then grinding or milling the solid phthalaldehyde with the salt to form particles or nanoparticles of the phthalaldehyde diluted in particles of the salt. The milling of the solid phthalaldehyde with the salt may both help to reduce the size of the particles, and mix or dilute the phthalaldehyde in the salt to help reduce caking, clumping, or other aggregation.

In another aspect, a solid containing phthalaldehyde plus salt, and any other optional ingredients, may first be prepared, and then ground into particles. The phthalaldehyde, salt, and any other optional ingredients may be dissolved into a solution. Then the solution may be dried to form the solid composition including the mixture of the phthalaldehyde, salt, and other optional ingredients. The solid composition may then be ground. Such homogeneous or nearly homogeneous incorporation of phthalaldehyde and salt into particles may facilitate disintegration and dissolution of the particles into solution. A specific example of a solid composition that may be prepared by such methods is shown in Example 24.

In spray drying, particles of phthalaldehyde, or particles of phthalaldehyde containing salt, may be formed. A method of preparing the particles, according to one embodiment, may include spray drying a solution containing dissolved phthalaldehyde to form particles containing the solid phthalaldehyde. Suitable approaches for spray drying are known in the arts. In a representative example of spray drying, a solution containing phthalaldehyde and optionally a salt may be prepared. Then, the solution may be sprayed into droplets of a fine mist or aerosol in an evaporation or drying chamber potentially containing an inert atmosphere. Then, the water or other solvent of the solution may be removed from the droplets in the evaporation chamber to form solid particles, or nanoparticles.

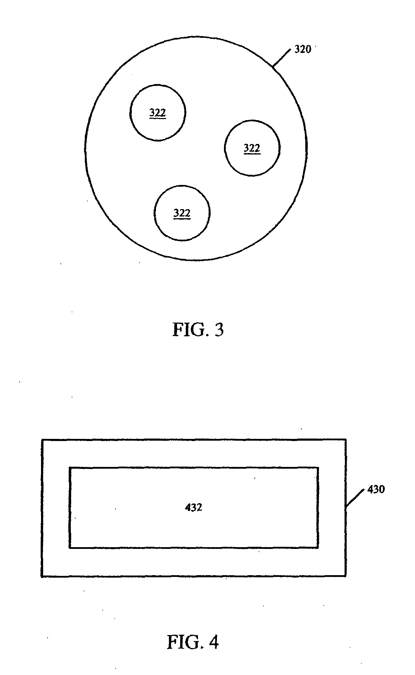

In one aspect, a dissolved salt, such as an enhancing salt, may be included in the solution that is spray dried to form particles containing a combination of the solid phthalaldehyde and the solid salt. Figure 3 shows a nano-sized or micron-sized particle containing phthalaldehyde 320 and at least one water-soluble salt 322, according to one embodiment of the invention. Suitable water soluble salts include the enhancing salts previously discussed, as well as other water-soluble salts, whether or not they are enhancing, such as sodium sulfate (Na₂SO₄), and combinations of such salts. Non-salt compounds such as starch, glucose, or cellulose may also optionally be employed, as long as they are soluble. The salt or non-salt may dissolve rapidly in water or another polar solvent and may facilitate dissolution of the particle. A specific example of a solid composition that may be prepared by such a method is disclosed in Example 25.

The size of the spray-dried particles generally depends on the size of the droplets, and the amount of the dissolved solids in the droplets. Generally, the smaller the droplets, and the smaller the amount of dissolved solids, the smaller the particles formed by spray drying. Other examples of forming particles or nanoparticles by spray drying are discussed in U.S. Patent Nos. 6,565,885; 6,451,349; and 6,001,336. Additionally, further background information on spray drying, if desired, is available in the Spray Drying Handbook, 4th Ed., written by Keith Masters, published by John Wiley & Sons, published in May 1985, ISBN: 0470201517.

The solid composition may be employed as a powder or shaped solid having a predetermined shape and size. Suitable shaped solids include, but are not limited to, blocks, tablets, capsules, flakes, and the like. The shaped solid may be formed by compression of phthalaldehyde and a diluent such as salt in a press or tablet press.. A conventional water-soluble binder material, such as those used in pharmaceutical tablets or laundry detergent tables, may be included to help enhance integrity of the shape. Alternatively, the shaped solid may be formed into various shapes by using molds. The molten liquid may be introduced into the mold, cooled, and therein solidified to form the shaped solid defined by the mold. The shaped solid may have a size that is sufficient to provide an appropriate amount or concentration of material, such as phthalaldehyde, in a predetermined volume of solution. The volume of solution may be a liter, a gallon, or a volume of a standard chamber (e.g., a hospital processing basin), for example. The salt of the shaped solid may serve as a disintegrating agent to help the solid to disintegrate once introduced into the solvent. Potential advantages of the shaped solid may include easier handling and improved control over solution concentration.

The solid composition may be placed in a water vapor or liquid impermeable or otherwise resistant container, such as a metal (for example aluminum), plastic laminated metal, or plastic pouch or bag, and sealed therein. The water resistant container may help to avoid entrance of water, or moisture, which could promote loss of phthalaldehyde due to the Cannizzaro reaction. An aluminum or other opaque material may be appropriate to block penetration of light and thereby help to prevent potential photochemical reactions, such as photodimerization of phthalaldehyde. An aluminum or other penetration resistant material may also be appropriate to help reduce the penetration of foreign substances into the solid composition. This may help to reduce a potential oxidation of phthalaldehyde (for example as shown below):

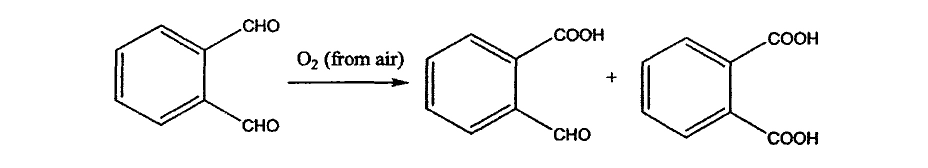

As other options, the pouch or other container may be filled with nitrogen, carbon dioxide, or other suitable inert gases. The inclusion of such inert gases may help to prevent penetration of moisture and may help to keep the composition dry. Nitrogen may help to prevent potential chemical reactions, if any, within the package. Carbon dioxide may react with trace amounts of hydroxide ion (OH⁻), which may be formed by reaction of water and bicarbonate, as follows:

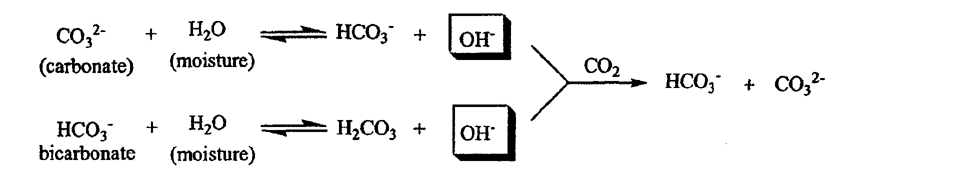

This may help to consume the water or moisture in the package. Such aspects are optional. The contained solid composition may then be distributed to a point of use, and stored until needed.

Figure 4 shows a solid composition 432 useful for preparing a germicidal solution sealed in a water-resistant container 430, according to one embodiment of the invention. The solid composition may include a shaped solid containing phthalaldehyde and an enhancer salt, such as a halide or bicarbonate salt.

A method of preparing a germicidal solution, according to one embodiment of the invention, may include opening a container, such as a water-resistant pouch or bag, removing a solid composition including solid salt and solid phthalaldehyde from the container, combining the solid composition with a solvent, such as water, and dissolving the solid composition in the solvent. Then, the germicidal solution so prepared may be used for disinfection, or sterilization.

### VIII. OPTIONAL COMPONENTS FOR COMPOSITION

The compositions disclosed herein may optionally contain chelating agents, corrosion inhibitors, surfactants, dyes, fragrances, and other desired components. The components may be employed in amounts appropriate to achieve the desired chelating, corrosion inhibition, coloring, or other effect.

Examples of suitable chelating agents that may be employed in the germicidal composition include, but are not limited to, BDTA (N,N'-1,4-butanediylbis[N-(carboxymethyl)] glycine), EDTA, various ionized forms of EDTA, EGTA (N"-ursodeoxycholyl-diethylenetriamine-N,N,N'-triacetic acid), PDTA (N,N'-1,3-propanediylbis[N-(carboxymethyl)) glycine), TTHA (3,6,9,12-Tetraazatetradecanedioic acid, 3,6,9,12-tetrakis(carboxymethyl)), trisodium HEDTA (N-[2[bis(carboxymethyl) amino]ethyl]-N-(2-hydroxyethyl)-glycine, trisodium salt), sometimes known as Versenol 120. Numerous other chelating agents known in the arts may also optionally be employed.

Examples of suitable corrosion inhibitors that may be employed in the germicidal composition include, but are not limited to, ascorbic acid, benzoic acid, benzoimidazole, citric acid, 1H-benzotriazole, 1-hydroxy-1H-benzotriazole, phosphate, phosphonic acid, pyridine, and sodium benzoate. Numerous other corrosion inhibitors known in the arts may also optionally be employed.

Examples of suitable dyes that may be employed in the germicidal composition include, but are not limited to, Blue 1 (Brilliant Blue FCF) if a bluish color is desired, D&C Green No. 5, D&C Green No. 6, and D&C Green No. 8, if a greenish color is desired, Yellow No. 5 if a yellowish color is desired, etc. Numerous other dyes known in the arts may also optionally be employed.

### IX. GERMICIDAL KITS

The inventors have developed germicidal containers and kits that may be used to contain, store, and distribute ingredients for preparing germicidal solutions. The kits may include multiple compartments, either in the same container or in different containers. The containers may include cans, tanks, bottles, boxes, bags, canisters, pouches, or other rigid or flexible containers known in the arts. In various aspects, the kits may provide phthalaldehyde in a solid composition to reduce losses due to the Cannizzaro reaction, or the kits may provide different compartments to separate phthalaldehyde from carbonates or other ingredients that may potentially interact negatively with the phthalaldehyde. Potential advantages of the kits include greater stability of the phthalaldehyde and potentially reduced transportation costs and storage space due to the elimination or reduction of liquid component.

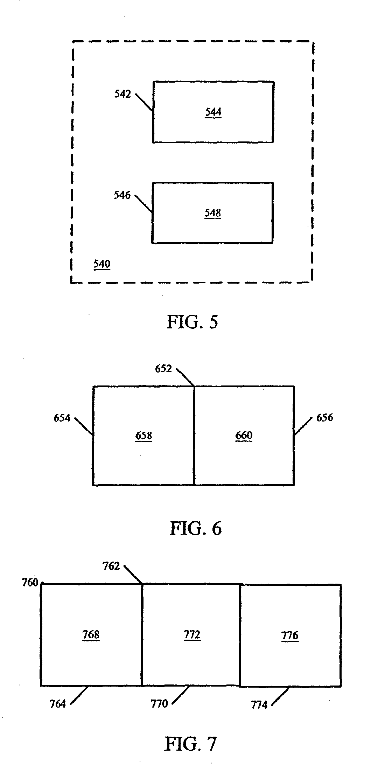

According to one embodiment of the invention, a kit for preparing a germicidal solution may include phthalaldehyde, an enhancer, and an optional solvent, wherein the phthalaldehyde, the enhancer, and the solvent are included in at least two compartments or containers. Figure 5 shows an exemplary germicidal kit 540 for preparing a germicidal solution, according to one embodiment of the invention. The kit includes a first container 542 containing a solid phthalaldehyde-containing composition 544. The solid composition may be similar to the other solid compositions discussed elsewhere herein. The illustrated kit also includes an optional second container 546 containing a solvent 548 to help dissolve the solid composition. The solvent may be combined with the solid composition, either in the first container, the second container, or another suitable container (for example a bucket or processing basin). It will be appreciated that the second container is not required and that solvent from another source, such as water from a tap, may also optionally be employed to dissolve the solid composition. In another aspect, phthalaldehyde may be included in the first container, and an enhancer for phthalaldehyde may be included in the second container. Other arrangements are contemplated. The phthalaldehyde, enhancer, and/or other chemicals, may be either liquid or solid. Further, the illustrated kit includes two separate containers and compartments, although a single container with two separate compartments may also optionally be employed.

In another embodiment of the invention, a kit may include two or more separate containers, or separate compartments of a single container, to separate phthalaldehyde from one or more other ingredients that may potentially react with or otherwise have an adverse affect on the phthalaldehyde. **Figure 6** shows a germicidal kit 650 to prepare a germicidal solution containing phthalaldehyde and an enhancer for the phthalaldehyde, or other chemical, according to one embodiment of the invention. The kit includes a multi-compartment container 652 having a first compartment 654 and a second compartment 656. A first composition 658 of the kit is contained in the first compartment, and a second composition 660 of the kit is contained in the second compartment. The first and the second compositions may include liquids or solids, as appropriate for the particular implementation. The first compartment and the second compartment are physically separated and distinct to completely separate the first composition from the second composition during storage. The container may include a first lid or opening to remove the first composition and a second lid or opening to remove the second composition.

The first composition may include phthalaldehyde. The phthalaldehyde may be provided as a dry solid or dissolved in water or an organic solvent. In the case of a solution, the solution may have a low or acidic pH sufficient to suppress the Cannizzaro reaction and help to improve the chemical stability of the phthalaldehyde. A pH adjuster, such as EDTA free acid, or another carboxylic acid, may be included in the first composition to help acidify the pH. Enough pH adjuster may be included to give a pH that is less than about 7.5, or less than about 6. In the case of a dry solid, the Cannizzaro reaction generally proceeds very slowly.

The second composition may include an enhancer for the phthalaldehyde, such as a halide salt, an alkali metal halide salt, a carbonate salt, a bicarbonate salt, etc. Other salt enhancers, such as phosphate, may also optionally be included, as well as optional pH adjusters (for example a buffer), chelating agents, corrosion inhibitors, surfactants, dyes, fragrances, and other desired components. In general, ingredients that may potentially have an adverse effect on phthalaldehyde may be included in the second composition. In the case of the composition being a solution, the pH of the second solution may be sufficiently high or alkaline that when combined with the first composition the resulting pH is from about 6 to 10, or from about 7.5 to 9. As discussed above, such pH generally enhance the germicidal efficacy of the phthalaldehyde. In this way the kit allows the phthalaldehyde in the first compartment may be isolated from an alkaline environment in the second compartment that may otherwise cause loss of phthalaldehyde due to the Cannizzaro reaction.

In one aspect, a method of using the kit to prepare a germicidal solution may include opening the container, and combining the first composition with the second composition. In one example, the contents of the compartments may be removed or poured serially into a processing basin or other container by a user or automated machine, such as an Automated Endoscope Reprocessor (AER). Then, depending on the desired phthalaldehyde concentration, water or another solvent may be introduced into the processing basin for dilution. Alternatively, the contents of the compartments may be combined within the container. In one embodiment of the invention, a container having a mechanism to automatically mix the first solution and the second solution upon opening of the container may be employed. Such containers are known in the arts. An exemplary container that is suitable is disclosed in U.S. Patent No. 5,540,326. This may also be achieved by forming an opening in the housing between the compartments, by rupture, tearing, opening a lid, etc. to combine the contents. As another option, a user or an automated machine, such as an AER, may flow water serially through the compartments in a predetermined order and then remove the water and contents to the processing basin. Once the germicidal solution of appropriate concentration is prepared in the processing basin, it may then be used for disinfection, sterilization, or both. Alternatively, the water or other solvent may be flowed through the compartments in parallel, either by the user or the automated machine.

In yet another example, a kit may include three separate containers each having a compartment, or three separate compartments of a single container, to separate ingredients that may potentially have an adverse affect on one another during a prolonged storage period. **Figure 7** shows an exemplary germicidal kit 760 including a container 762 having a first compartment 764 containing a solvent 768, a second compartment 770 containing a solid phthalaldehyde-containing composition 772, and a third compartment 774 containing an enhancer or other chemical to be employed with the phthalaldehyde 776, according to one embodiment of the invention. The practitioner or an automated machine, such as an AER, may combine the contents of the containers or compartments. In one aspect, the contents may be combined in a predetermined order. For example, the automated machine may first autonomously combine the solvent of the first compartment or container with the phthalaldehyde of the second compartment or container. In the case of a multiple compartment container this may include forming an opening in a wall between the compartments. Then, the machine may combine the solvent-phthalaldehyde solution with the enhancer or other chemical of the third compartment or container. Then, the machine may introduce the resulting solution into a processing basin. As another option, in the case of the illustrated multiple-compartment container, the machine may flow water serially through the compartments in the predetermined order to form the germicidal solution.

In yet another embodiment of the invention, a container or compartment having a heating capability may be used to store and heat a solid phthalaldehyde composition. The heating capability may be used to heat the solid phthalaldehyde composition to a temperature greater than an ambient temperature to facilitate dissolution of the phthalaldehyde into a germicidal solution. In one aspect, the solid phthalaldehyde composition is heated to a melting point temperature of the phthalaldehyde to melt the phthalaldehyde to form a liquid that may readily be dissolved in solvent or water. Suitable heating capabilities include, but are not limited to, thermally conductive materials or surfaces that may be used to transfer heat into the interior of the container or compartment, electrical resistance heaters, exothermic reaction heaters, and other heaters known in the arts. If desired, the container or compartment having the heating capability may be included in a kit with other containers or compartments described herein.

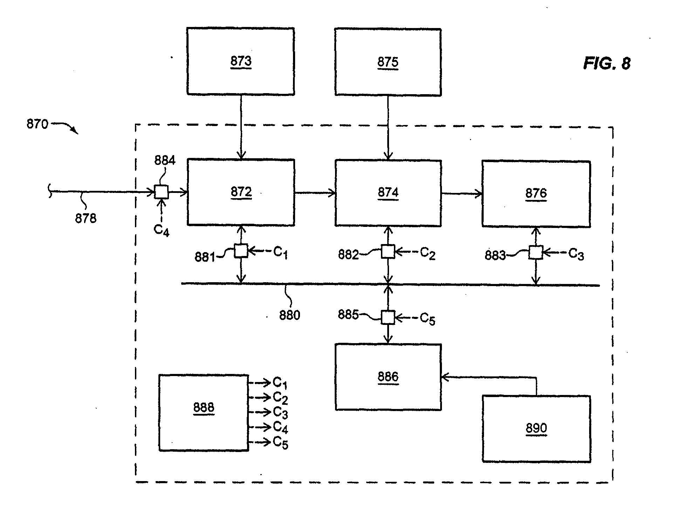

A germicidal solution preparation apparatus may be used to prepare a germicidal solution. Figure 8 shows a germicidal solution preparation apparatus 870, according to one embodiment of the invention. The apparatus includes a first port 872 to receive a first solution preparation composition 873, and an optional second port 874 to receive a second solution preparation composition 875. If desired, other optional ports, such as a third optional port, and a fourth optional port, may be included. In one aspect, three ports may be included to prepare a germicidal solution from a first discrete composition including phthalaldehyde, a second discrete composition including an enhancer or other chemical to be employed with phthalaldehyde, and a third discrete composition including a solvent. A practitioner may provide the first and the second compositions to the appropriate ports. For example, the practitioner may pour the compositions into the ports or couple the containers or compartments with the ports. In one aspect, the first composition may include a phthalaldehyde-containing composition, and the second composition may include a solvent or an efficacy enhancer for phthalaldehyde. The apparatus may include feedback control mechanisms to provide the compositions from the ports. If desired, one or more of the ports may include heating capabilities, such as a heater, to facilitate dissolution of, or melt, a composition. For example, a port may include a heater to melt phthalaldehyde.

The apparatus also includes a source of water 878, a germicidal solution holding chamber 876 to hold a prepared germicidal solution, germicidal solution preparation logic 888 to control the preparation of the germicidal solution from the compositions and the water, and a processing chamber 886 to carry out disinfection or sterilization with the prepared germicidal solution. The source of water is optional and may include tap water or a de-ionized water line. The ports, the chambers, and the water line are each fluidically coupled with a piping system 880 of the apparatus. The piping system generally provides a fluid pathway for movement of fluids around the apparatus.

The solution preparation logic 888 provides the logic to prepare the germicidal solution from the compositions and the water. The may include hardware, software, or a combination, and may specify flows, times, etc. to achieve the appropriate mixing of the compositions with the water. In the illustrated embodiment, the logic provides control signals C1-C5 to controllers 881-885, such as valves, positioned on lines connecting the ports, chambers, and the source of the water with the piping system. The logic may employ the control signals to introduce the compositions and the water into the holding chamber. In one aspect, the controls may provide that the water flushes the first composition into the holding chamber, then flushes the second composition into the holding chamber, then adds appropriate amounts of water to the holding chamber to achieve the desired dilution of phthalaldehyde. The control signals may also control the introduction of the prepared germicidal solution from the holding chamber to the processing chamber. At this point the germicidal solution may be used for disinfection or sterilization. In one aspect, the solution may be used for disinfection or sterilization of medical devices. For example, in the case of the apparatus including an automated endoscope reprocessor, a practitioner may position an endoscope in the apparatus. The apparatus may include a manifold and connectors to flow fluid into channels of the endoscope and contact a surface of the endoscope with the solution in order to disinfect or sterilize the surface.

Since the germicidal solution is prepared by the apparatus instead of being pre-prepared with quality control and testing in a manufacturing environment, it may be appropriate to include optional capability for the apparatus to interrogate or test the prepared germicidal solution prior to use. In one aspect, the apparatus may include a germicidal solution interrogation or test system 890 to interrogate or test the germicidal solution prior to use for disinfection or sterilization. For example, the apparatus may include an ultraviolet spectroscopy system, or other concentration determination instrumentation, to determine the concentration of phthalaldehyde in the prepared germicidal solution. The determination of OPA concentration may be determined directly or by determining the concentration of a reaction product of OPA with another chemical such as glycine. The concentration may be determined in the holding chamber, the processing chamber (as shown), or inline in the piping system. Other testing systems based on test strips or the like may also optionally be employed.

### X. EXAMPLES

Having been generally described, the following examples are given as particular embodiments of the invention, to illustrate some of the properties and demonstrate the practical advantages thereof, and to allow one skilled in the art to utilize the invention. It is understood that these examples are to be construed as merely illustrative, and not limiting. For example, the experiments were conducted at a concentration of 0.3% by weight phthalaldehyde, although this concentration is not required. Lower concentrations down to about 0.025% by weight may be employed with longer exposure times or higher temperatures, or higher concentrations up to about 2% may be employed with shorter exposure times.

As another example, the experiments were conducted at a temperature of approximately 20°C (room temperature) to avoid heating or cooling, although this particular temperature is not required. In general, the disinfection or sterilization may be carried out at a temperature between about 10°C to 80°C, or especially between about 20°C to 60°C. Temperatures between about 20°C to 60°C may be achieved with minor heating, or by using heated water. Generally a higher temperature improves the germicidal efficacy.

As yet another example, the experiments are conducted with highly resistant *Bacillus subtilis* spores, although this is not required. The compositions are generally able to kill less resistant microbes, such as mycobacteria, nonlipid or small viruses, or fungi, in shorter times or with lower concentrations or temperatures; even more resistant microbes, may potentially be killed with longer exposure times, higher concentrations, or higher temperatures.

### Example 1

This example demonstrates how to prepare a 0.3% (w/v) phthalaldehyde germicidal solution. The solution was prepared by dissolving 0.3 g phthalaldehyde in de-ionized water, and then adding additional water make 100 milliliters (mL) solution. The phthalaldehyde was obtained from DSM Chemie Linz, located at St. Peter Strasse 25, P.O. Box 296, A-4021 Linz/Austria. When appropriate, the ingredients listed in the tables below were further included in phthalaldehyde solution in amounts appropriate to achieve solutions with the concentrations specified in the tables.

### Example 2

This example demonstrates the well-known spore suspension test procedure used to make the determination of effectiveness. In this test method, 9 mL of the germicide to be tested is placed in a tube, put into a water bath and allowed to come to the desired temperature. 1 mL of the test organism, including at least 7 logs/mL of *Bacillus subtilis* spores, is added to the 9. mL of the germicide to be tested.. The dilution resulted in at least 6 logs/mL of the spores in the mixture. It will be appreciated by those skilled in the art that other concentrations may be utilized by proper dilution and accounting.

At appropriate time intervals, 1 mL aliquots of the germicide-cell suspension were removed and added directly into 9 mL of a 1% glycine solution (neutralizer) and mixed thoroughly to neutralize the germicide in the transferred suspension. The glycine solution was prepared from solid glycine, which is available from VWR Scientific Products, among others. The above-identified 10 mL neutralized solution was then poured through a membrane filter having an average pore size of 0.45 micrometers. The filter was then rinsed twice with at least 150 mL of the 1% glycine solution per rinse. The filter was then placed on an agar plate and incubated for at least two days at 37°C. In the above procedure, if dilution was needed, then the 1 mL germicidal-cell suspension was diluted in 99 mL of a phosphate buffer before addition to the 9 mL of the 1% glycine solution. The phosphate buffer was DiLu-LoK™ Butterfield's Phosphate Buffer, available from Hardy Diagnostics, of Santa Maria, California.

The surviving colonies were then counted. The data is plotted as S/Sₒ vs. time. So is the initial count of the spores in the above 10 mL solution which is at least 10⁶ spores/mL, and S is the surviving spores from the above filter on the agar plate. The results of the experiments were presented in terms of log reductions. Log reduction is the difference between log(Sₒ) and log(S). As an example, if log(Sₒ) = 6.2, and if there were 100 survivors, then the log(S) =2, and the log reduction was reported as 4.2.

### Example 3

A solution including 1000 mM sodium fluoride (NaF) without phthalaldehyde, and several germicidal solutions containing from 100 mM to 1000 mM NaF with 0.3% phthalaldehyde, were tested to determine their effectiveness at killing *Bacillus subtilis* spores. The solutions were tested at a temperature of 20°C and at exposure times of 4, 8, and 24 hours. The differences in pH are due to the chemical additions shown without further pH control. The results are shown in Table 1.

**Table 1**

| [OPA] | [NaF] | pH | Log Reduction/mL (20°C) | | |
|---|---|---|---|---|---|
| | | | 4 hr | 8 hr | 24 hr |
| 0% | 1000mM | 7.6 | Not Tested | 0.0 | 0.0 |
| 0.3% | 0mM | 7.0 | 0.5 | 0.6 | 2.9 |
| 0.3% | 100mM | 7.3 | 0.9. | 1.7 | Total Kill |
| | 200mM | 7.5 | 3.8 | 4.6. | Total Kill |
| | 400mM | 7.6 | 4.7 | Total Kill | Total Kill |
| | 800mM | 7.7 | >6.0 | Not Tested | Not Tested |
| | 1000mM | 7.7 | Total Kill | Not Tested | Not Tested |

The results show that NaF enhances the germicidal efficacy of phthalaldehyde. The results also show that a higher NaF concentration, at least over the range between 100 to 1000 mM, generally provides greater enhancement. For the 0.3% phthalaldehyde solutions tested, the 1000 mM NaF solution was effective at achieving a total kill within only 4 hours, the 400 mM NaF solution was effective at achieving a total kill within 8 hours, and the 100 and 200 mM NaF solutions were effective at achieving a total kill of the spores within 24 hours. The non-phthalaldehyde solution containing 1000 mM of NaF was unable to achieve greater than a 0.0 log reduction of spores within 24 hours. This indicates that the 1000 mM NaF is practically non-germicidal with respect to the spores.

### Example 4

Several solutions that each included a sodium halide salt, namely sodium fluoride (NaF), sodium chloride (NaCI), sodium bromide (NaBr), and sodium iodide (NaI), were tested, both with and without phthalaldehyde, to determine their effectiveness at killing *Bacillus subtilis* spores. A first set of solutions included the sodium halide salts at a 1000 mM concentration, but lacked phthalaldehyde. A second set of solutions the sodium halide salts at a 1000 mM concentration, and included 0.3% phthalaldehyde. The solutions were tested at a temperature of 20°C and at exposure times of 4, 8, and 24 hours. The differences in pH are due to the chemical additions shown without further pH control. The results are shown in Table 2.

**Table 2**

| [OPA] | 1000mM of [NaX] | pH | Log Reduction/mL (20°C) | | |
|---|---|---|---|---|---|
| | | | 4 hr | 8 hr | 24 hr |
| 0% | NaF | 7.6 | Not tested | 0.0 | 0.0 |
| | NaCl | 7.2 | Not tested | Not tested | 0.2 |
| | NaBr | 6.2 | 0.0 | Not tested | 0.1 |
| | NaI | 8.3 | 0.0 | 0.0 | 0.1 |
| 0.3% | 0mM | 7.0 | 0.5 | 0.6 | 2.9 |
| 0.3% | NaF | 7.7 | Total kill | Not tested | Not tested |
| | NaCl | 5.9 | Not tested | 3.3 | Total kill |
| | NaBr | 6.5 | 1.9 | Total kill | Total kill |
| | NaI | 7.2 | 2.8 | Total kill | Total kill |

The results show that each of the sodium halides NaF, NaCl, NaBr, and NaI enhance the germicidal efficacy of phthalaldehyde. A 0.3% phthalaldehyde solution alone is generally able to achieve a log reduction of only about 0.5 in 4 hours, 0.6 in 8 hours, and 2.9 in 24 hours. However, the 0.3% phthalaldehyde solutions containing the sodium halides were able to achieve significantly greater log reductions. In particular, the 0.3% phthalaldehyde solution containing NaF was effective at achieving a total kill in only 4 hours, the solutions containing NaBr and NaI were effective at achieving a total kill in 8 hours, and the solution containing NaCl was effective at achieving a log reduction of 3.3 in 8 hours. This, coupled with the data showing that the sodium halide solutions that lacked phthalaldehyde had only negligible log reductions in 24 hours (less than 0.2 log reductions), indicates that the sodium halides enhance the germicidal efficacy of phthalaldehyde. The data also seem to indicate that NaF enhances the germicidal efficacy more than the other sodium halides, and that NaBr and NaI enhance the efficacy better than NaCl.

### Example 5

Several solutions that each included an inorganic fluoride salt, namely potassium fluoride (KF), or lithium fluoride (LiF), were tested, both with and without phthalaldehyde, to determine their effectiveness at killing *Bacillus subtilis* spores. A first set of solutions included the fluoride salts without phthalaldehyde. A second set of solutions included the fluoride salts and 0.3% phthalaldehyde. The fluoride salts were employed at a concentration sufficient to achieve 1000 mM of the fluoride ion (F). The solutions were tested at a temperature of 20°C and at exposure times of 4, 8, and 24 hours. The differences in pH are due to the chemical additions shown without further pH control. The results are shown in Table 3.

**Table 3**

| [OPA] | Concentration of [F-] | pH | Log Reduction/mL (20°C) | | |
|---|---|---|---|---|---|
| | | | 4 hr | 8 hr | 24 hr |
| 0% | 1000mM of KF | 7.9 | Not tested | 0.0 | 0.1. |
| | 100mM of LiF | 9.5 | Not tested | Not tested | 0.0 |
| 0.3% | 0mM | 7.0 | 0.5 | 0.6 | 2.9. |
| 0.3% | 1000mM of KF | 8.0 | 5.8 | Not tested | > 6.0 |
| | 100mM of LiF | 9.1 | 1.8 | 3.4 | >6.0 |

The results indicate that the alkali metal fluoride salts KF and LiF enhance the germicidal efficacy of phthalaldehyde. A 0.3% phthalaldehyde solution alone is generally able to achieve a log reduction of only about 0.5 in 4 hours, 0.6 in 8 hours, and 2.9 in 24 hours. However, the 0.3% phthalaldehyde solutions containing the KF and LiF fluoride salts were able to achieve significantly greater log reductions in 4 hours and 8 hours. In particular, the 0.3% phthalaldehyde solution containing KF was effective at achieving a log reduction of 5.8 in only 4 hours, and the solutions containing LiF was effective at achieving a log reduction of 1.8 in 4 hours and 3.4 in 8 hours. In contrast, the fluoride salt solutions that lacked phthalaldehyde had only negligible log reductions in 24 hours (less than 0.3 log reductions), indicating a synergy or enhancement between the alkali metal fluoride salts and phthalaldehyde.

### Example 6A

Several solutions including 0.3% phthalaldehyde and 1000 mM potassium fluoride (KF) were tested at a range of pH from 6.6 to 10.1 to determine their effectiveness at killing *Bacillus subtilis* spores. The solutions were tested at a temperature of 20°C and at exposure times of 4, 8, and 24 hours. The pH were adjusted by adding NaOH. The results are shown in Table 4A.

**Table 4A**

| [OPA] | [KF] | pH | Log Reduction/mL (20°C) | | |
|---|---|---|---|---|---|
| | | | 4 hr | 8 hr | 24 hr |
| 0.3% | 0mM | 7.0 | 0.5 | 0.6 | 2.9 |
| 0.3% | 1000mM KF | 6.6 | 2.0 | Not tested | Total kill |
| | | 7.0 | 3.5 | Not tested | Total kill |
| | | 8.0 | 5.5 | Not tested | Total kill |
| | | 9.0 | >6.0 | Not tested | Total kill |
| | | 10.1 | Total kill | Not tested | Total kill |

The results show that increased alkalinity, or a higher pH, generally enhances the germicidal efficacy of a phthalaldehyde solution including an alkali metal halide salt, such as potassium fluoride, at least over the pH range from 6.6 to 10.1. The results also show that a 0.3% phthalaldehyde solution including 1000 mM KF is effective to achieve a total kill of the spores within 24 hours over the pH range of 6.6 to 10.1. At a pH of 10.1, the solution was able to achieve a total kill in only 4 hours.

### Example 6B

Solutions including 0.3% phthalaldehyde or 2.4% glutaraldehyde were tested, both with and without the presence of alkali metal halide salts to determine their effectiveness at killing *Bacillus subtilis* spores. Glutaraldehyde is non-aromatic dialdehyde. The particular alkali metal halide salts tested included 1000mM KF, 1000mM KI, and a mixture of 1000mM KF and 1000mM KI. Control solutions with the same concentrations of halide salts were also tested. The tests were conducted at a temperature of 20°C, at an exposure time of 3 hours, and at a pH of 8. The pH was due to the chemical additions. without further pH control. The results are shown in Table 4B.

**Table 4B**

| [KF] | [KI] | [OPA] | [Glutaraldehyde] | Log Reduction/mL (3 hr, 200°C, pH=8) |
|---|---|---|---|---|
| 0 | 0 | 0.3% | 0 | 0.10 |
| 0 | 0 | 0 | 2.4% | <1.1 |
| 1000mM | 0 | 0 | 0 | 0.0 |
| 0 | 1000mM | 0 | 0 | 0.0 |
| 1000mM | 1000mM | 0 | 0 | 0.0 |
| 1000mM | 0 | 0 | 2.4% | 4.2 |
| 1000mM | 1000mM | 0 | 2.4% | Total kill |
| 1000mM | 1000mM | 0.3% | 0 | Total kill |

The results indicate that alkali metal halide salts enhance the germicidal efficacy of glutaraldehyde. A 2.4% glutaraldehyde solution without alkali metal halide salts is able to achieve a log reduction of <1.1 in 3 hours. However, when 1000mM KF is included along with the 2.4% glutaraldehyde, a much higher log reduction of 4.2 is achieved. Likewise, when 1000mM KF and 1000mM KI are included, along with the 2.4% glutaraldehyde, a total kill is achieved in only three hours. These results may indicate a general capability of halide salts to enhance the efficacy of dialdehyde germicides, or potentially germicides in general.

### Example 7

Several solutions including 0.3% phthalaldehyde and either 0 or 250 mM of various salts (sodium chloride (NaCl), sodium bromide (NaBr), sodium iodide (NaI), sodium sulfate (Na₂SO₄), KH₂PO₄/K₂HPO₄, and EDTA·3Na) were tested both with and without 400 mM of sodium fluoride (NaF) to determine their effectiveness at killing *Bacillus subtilis* spores. The solutions were tested at a temperature of 20°C and an exposure time of 4 hours. The differences in pH are due to the chemical additions shown without further pH control. The results are shown in Table 5.

**Table 5**

| [OPA] | Salt (250mM) | [NaF], 0mM | | [NaF], 400mM | |
|---|---|---|---|---|---|
| | | pH | Log Reduction/mL (20°C, 4hrs) | pH | Log Reduction/mL (20°C, 4hrs) |
| 0.3% | None | 7.9 | 0.4 | 7.6 | 4.7 |
| | NaCl | 4.8 | 0.4 | 7.6 | 5.6 |
| | NaBr | 4.8 | 0.3 | 7.7 | 5.9 |
| | NaI | 7.2 | 0.5 | 7.7 | 5.9 |
| | Na₂SO₄ | 5.9 | 0.5 | 7.8 | >6.0 |

The results show that NaCl, NaBr, NaI, and Na₂SO₄ enhance the germicidal efficacy of a solution including phthalaldehyde and NaF. The log reductions 5.6, 5.9, 5.9, and >6.0 are each significantly larger than the log reduction 4.7 observed when the salts NaCl, NaBr, NaI, and Na₂SO₄, respectively, were not included.

### Example 8

Several germicidal solutions including 0.3% phthalaldehyde and 1000 mM a sodium or potassium halide were tested to determine their material compatibility with stainless steel and DuPont™ Teflon® brand polytetrafluoroethylene. These materials are commonly employed in endoscopes and other medical devices. The tests were performed at a temperature of 20°C, and an exposure time of 72 hours. The compatibility was judged by visual examination. The results are shown in Table 6.

**Table 6**

| | 0.3% OPA + 1000 mM (NaX or KX) at 20°C for 72 hr | |
|---|---|---|
| NaX or KX | Stainless Steel | Teflon |
| NaF | Compatible | Compatible |
| NaCl | Compatible | Compatible |
| NaBr | Compatible | Compatible |
| NaI | Compatible | Compatible |
| KF | Compatible | Compatible |
| KCl | Compatible | Compatible |
| KBr | Compatible | Compatible |
| KI | Compatible | Compatible |

The results show that all solutions are compatible with stainless steel and Teflon.

### Example 9

A series of germicidal solutions containing from 0 mM (millimolar) to 500 mM of sodium bicarbonate (NaHCO₃), or either 0 mM or 250 mM potassium carbonate (K₂CO₃) were tested to determine their effectiveness at killing *Bacillus subtilis* spores, over several exposure times from 2 to 24 hours, at a temperature of 20°C. The results are shown in Table 7.

**Table 7**

| [OPA] | [NaHCO₃] mM | [K₂CO₃] mM | pH | Log Reductions/mL (20°C) | | | | |
|---|---|---|---|---|---|---|---|---|
| | | | | 2hr | 4hr | 6hr | 8hr | 24hr |
| 0.3% | 0 | 0 | 7.7 | 0.4 | 0.5 | 0.7 | 0.6 | 2.9 |
| | 17 | 0 | 8.6 | 0.4 | 0.6 | 0.7 | 0.7 | 4.8 |
| | 63 | 0 | 8.6 | 0.7 | 1.5 | 2.4 | 3.5 | Total kill |
| | 125 | 0 | 8.7 | 1.2 | 3.5 | 5.1 | 5.6 | Total kill |
| | 250 | 0 | 8.7 | 3.4 | 5.2 | 5.7 | > 6.0 | Total kill |
| | 0 | 250 | 8.4 | Not tested | _{4.7} | Not tested | Not tested | Not tested |
| | 500 | 0 | 8.4 | 3.0 | 5.0 | 5.8 | > 6.0 | Total kill |

The results show that the killing of the spores, as evidenced by the log reductions, is enhanced by carbonate and bicarbonate. The enhancement increases with increasing bicarbonate concentration. A concentration of sodium bicarbonate of 63 mM or higher is sufficient to achieve sterilization as represented by a total kill of all spores within 24 hours.

### Example 10

Solutions containing 250 mM sodium bicarbonate (NaHCO₃) were tested to determine their effectiveness at killing *Bacillus subtilis* spores at a pH of 8.2, 9.2, and 10.3, at a temperature of 20°C, and at exposure time of 4 hours. The pH values were maintained by adding either HCl or NaOH to achieve the listed pH. The results are shown in Table 8.

**Table 8**

| OPA | NaHCO₃ | Temperature | pH | Log Reductions/mL With 4 hr exposure |
|---|---|---|---|---|
| 0.3% | 250mM | 20°C | 8.2 | 5.1 |
| | | | 9.2 | 5.4 |
| | | | 10.3 | 5.7 |

The results show that a higher or more alkaline pH, at least over the range from 8.2 to 10.3, generally enhances the killing of spores by a solution containing phthalaldehyde and bicarbonate.

### Example 11

Several germicidal solutions containing 63 mM phosphate and bicarbonate or carbonate from different salts were tested to determine their effectiveness at killing *Bacillus subtilis* spores. The solutions were tested at a temperature of 20°C, and exposure times of 2 and 4 hours. The differences in pH are due to the chemical additions shown without further pH control. The results are shown in Table 9.

**Table 9**

| [OPA] | Carbonate Source | Phosphate | pH | Log Reduction/mL (20°C) | |
|---|---|---|---|---|---|
| | | | | 2hr | 4 hr |
| 0.3% | 250mM NaHCO₃ | 63mM | 8.4 | 2.1 | Total kill |
| | 200mM NaCl | | | | |
| | 30g EDTA-3Na | | | | |
| | 125mM LiCO₃ | | 8.6 | 1.9 | Total kill |
| | 125mM K₂CO₃ | | 8.3. | 1.9 | Total kill |

The results show that each of the three solutions achieved a total kill in 4 hours, or less. The results also confirm that carbonates derived from different alkali salts provide suitable enhancers.

### Example 12

A germicidal solution containing no carbonate, a germicidal solution containing 125mM of sodium bicarbonate (NaHCO₃), and a germicidal solution containing sodium hydroxide (NaOH) solution saturated with carbonation by purging at atmospheric pressure were tested to determine their effectiveness at killing *Bacillus subtilis* spores. The solutions were tested at a temperature of 20°C, and at exposure times of 4 and 24 hours. The differences in pH are due to the chemical additions shown without further pH control. The results are shown in Table 10.

**Table 10**

| [OPA] | Carbonate Source | pH | Log Reductions/mL (20°C) | |
|---|---|---|---|---|
| | | | 4 hr | 24 hr |
| 0.3% | None | 7.0 | 1.5 | 2.9 |
| | 125mM of NaHCO₃ | 8.6 | 3.3 | Total kill |
| | 125mM of NaOH solution purged with CO₂ gas until pH stabilized | 7.6 | 2.5 | Total kill |

The results show that the phthalaldehyde solutions containing bicarbonate are able to achieve total kill of *Bacillus subtilis* spores within 24 hours. The phthalaldehyde solution without the bicarbonate or carbon dioxide did not achieve total kill, and only achieved a log reduction of 2.9 within 24 hours. The results also show that a carbonation of an alkaline solution provides a suitable source of enhancing carbonates.

### Example 13

Germicidal solutions containing either 0 mM or 63 mM phosphate, and either 0 mM, 125 mM, or 250 mM sodium bicarbonate (NaHCO₃) were tested to determine their effectiveness at killing *Bacillus subtilis* spores.. The solutions were tested at a temperature of 20°C, an exposure time of 4 hours. The pH was between 7.9 and 8.4 and was due to the chemical additions shown without further pH control. The results are shown in Table 11.

**Table 11**

| [OPA] | [NaHCO3] | [Phosphate], 0mM | | [Phosphate], 63mM | |
|---|---|---|---|---|---|
| | | pH | Log Reduction/mL (20°C, 4hrs) | pH | Log Reduction/mL (20°C, 4hrs) |
| 0.3% | 0mM | 7.9 | 0.4 | 8.3 | 0.7 |
| | 125mM | 8.3 | 3.0 | 8.4 | 5.3 |
| | 250mM | 8.3 | 4.0 | 8.4 | 5.5 |

The results show that phosphate enhances the killing of spores by phthalaldehyde when employed with bicarbonate. The phosphate appears to provide a very slight enhancement without bicarbonate. The results also confirm that sodium bicarbonate enhances the killing of phthalaldehyde, and that the enhancement generally increases with concentration over the tested range of 0 mM to 250 mM.

### Example 14

A germicidal solution containing no potassium halides, and several germicidal solutions containing 100 mM of one of potassium chloride (KCl), potassium bromide (KBr), potassium iodide (KI), or potassium fluoride (KF), were tested, both with and without a concentration of 63 mM sodium bicarbonate (NaHCO₃), to determine their effectiveness at killing *Bacillus subtilis* spores. The solutions were tested at a temperature of 20°C and an exposure time of 4 hours. The differences in pH are due to the chemical additions shown without further pH control. The results are shown in Table 12.

**Table 12**

| [OPA] | [KX] (100Mm) | [NaHCO₃], 0mM | | [NaHCO₃], 63mM | |
|---|---|---|---|---|---|
| | | pH | Reduction/mL (20°C, 4hrs) | pH | log Reduction/mL (20°C, 4hrs) |
| 0.3% | No KX | 7.7 | 0.5 | 8.6 | 1.5 |
| | KCl | 4.9 | 0.3 | 8.6 | 4.8 |
| | KBr | 6.9 | 0.5 | 8.7 | 4.9 |
| | KI | 6.9 | 0.5 | 8.5 | 4.7 |
| | KF | 8.0 | 0.8 | 8.4 | 4.9 |

The results show that the potassium halides enhance the killing of spores by phthalaldehyde when employed with bicarbonate.

### Example 15

A germicidal solution containing no sodium halides, and several germicidal solutions containing 100 mM of one of sodium chloride (NaCl), sodium bromide (NaBr), sodium iodide (NaI), or sodium fluoride (NaF), were tested, both with and without a concentration of 63 mM sodium bicarbonate (NaHCO₃), to determine their effectiveness at killing *Bacillus subtilis* spores. The solutions were tested at a temperature of 20°C and an exposure time of 4 hours. The differences in pH are due to the chemical additions shown without further pH control. The results are shown in Table 13.

**Table 13**

| [OPA] | [NaX] (100mM) | [NaHCO₃], 0mM | | [NaHCO₃], 63mM | |
|---|---|---|---|---|---|
| | | pH | Log Reduction/mL (20°C, 4hrs) | pH | Log Reduction/mL (20°C, 4hrs) |
| 0.3% | No NaX | 7.7 | 0.5 | 8.6 | 1.5 |
| | NaCl | 6.8 | 0.4 | 8.4 | 2.3 |
| | NaBr | 6.6 | 1.3 | 8.5 | 2.2 |
| | NaI | 7.0 | 1.5 | 8.5 | 2.1 |
| | NaF | 7.3 | 0.9 | 8.7 | 2.5 |

The results show that even at low concentrations, several of the sodium halides, namely NaF, NaBr, and NaI, may enhance the killing of spores by phthalaldehyde, when employed without bicarbonate. Also, several of the sodium halides, namely NaCl and NaF, may enhance the killing of spores by phthalaldehyde, when employed with bicarbonate. Still further, the results show that either individually or combined the sodium halide salts and the bicarbonate enhance the efficacy of the phthalaldehyde.

### Example 16

Germicidal solutions containing from 0 mM to 250 mM of sodium chloride (NaCl) were tested with or without 63 mM sodium bicarbonate (NaHCO₃) to determine their effectiveness at killing *Bacillus subtilis* spores. The solutions were tested at a temperature of 20°C and an exposure time of 4 hours. The differences in pH are due to the chemical additions shown without further pH control. The results are shown in Table 14.

**Table 14**

| [OPA] | [NaCl] | [NaHCO₃], 0mM | | [NaHCO₃], 63mM | |
|---|---|---|---|---|---|
| | | pH | Log Reduction/mL (20°C, 4hrs) | pH | Log Reduction/mL (20°C, 4hrs) |
| 0.3% | 0 mM | 7.7 | 0.5 | 8.6 | 1.5 |
| | 50mM | | Not tested | 8.4 | 1.5 |
| | 100mM | | Not tested | 8.4 | 1.9 |
| | 200 mM | | Not tested | 8.4 | 2.9 |
| | 250 mM | 6.8 | 0.4 | | Not tested |

The results show that NaCl enhances the killing of spores by phthalaldehyde when employed with bicarbonate. The enhancement begins to become noticeable at a concentration of from 50 to 100 mM, and the enhancement increases with concentration to at least 200 mM. The results also show that, under the test conditions, NaCl does not enhance killing when employed without bicarbonate.

### Example 17

A germicidal solution containing no polyalkylammonium halides, and several germicidal solutions containing 200 mM of one of Bu₄NF, Bu₄NCl, Bu₄NBr, or Bu₄Nl were tested, both with and without a concentration of 63 mM sodium bicarbonate (NaHCO₃), to determine their effectiveness at killing *Bacillus subtilis* spores.. The solutions were tested at a temperature of 20°C and an exposure time of 4 hours. The differences in pH are due to the chemical additions shown without further pH control. The results are shown in Table 15.

**Table 15**

| [OPA] | [Bu₄NX] (200mM) | [NaHCO₃], 0mM | | [NaHCO₃], 63mM | |
|---|---|---|---|---|---|
| | | pH | Log Reduction/mL (20°C, 4hrs) | pH | Log Reduction/mL (20°C, 4hrs) |
| 0.3% | No Bu₄NX | 7.0 | 0.5 | 8.6 | 1.4 |
| | Bu₄NF | 6.7 | 0.5 | 8.4 | 2.8 |
| | Bu₄NCl | 6.7 | 0.4 | 8.5 | 3.9 |
| | Bu₄NBr | 6.7 | 0.5 | 8.4 | 3.9 |
| | Bu₄NI With 57mM | 6.7 | 0.4 | 8.7 | 2.9 |

The results show that the polyalkylammonium halides enhance the killing of spores by phthalaldehyde when employed with bicarbonate. Bu₄NCl, and Bu₄NBr appear to provide slightly greater enhancement than Bu₄NF and Bu₄NI under the conditions tested. Note that the concentration of Bu₄NI was 57 mM, which is the solubility.

### Example 18

An aqueous germicidal solution having the concentrations listed in Table 16 was prepared and tested to determine its effectiveness at killing *Bacillus subtilis* spores. The tests were conducted at a pH of about 7.5, a temperature of 20°C, and an exposure time of 4 hours. The results indicated that the solution was effective to achieve a total kill of the spores in 4 hours.

**Table 16**

| Component | Concentration |
|---|---|
| Phthalaldehyde | 0.3% (w/v) |
| NaF | 900 mM |
| EDTA·2Na | 5 mM |
| EDTA·4Na | 5 mM |
| Water | Remainder |

### Example 19

An aqueous germicidal solution having the concentrations listed in Table 17 was prepared and tested to determine its effectiveness at killing *Bacillus subtilis* spores. The tests were conducted at a pH of about 7.5, a temperature of 20°C, and an exposure time of 4 hours. The results indicated that the solution was effective to achieve a total kill of the spores in 4 hours.

**Table 17**

| Component | Concentration |
|---|---|
| Phthalaldehyde | 0.3% (w/v) |
| KF | 1000 mM |
| K₂HPO₄ | 30mM |
| KH₂PO₄ | 10mM |
| EDTA·3Na | 10mM |
| Water | Remainder |

### Example 20

An aqueous germicidal solution having the concentrations listed in Table 18 was prepared and tested to determine its effectiveness at killing *Bacillus subtilis* spores. The tests were conducted at a pH of about 7, a temperature of 20°C, and an exposure time of 4 hours. The results indicated that the solution was effective to achieve a total kill of the spores in 4 hours.

**Table 18**

| Component | Concentration |
|---|---|
| Phthalaldehyde | 0.55% (w/v) |
| KF | 1000 mM |
| K₂HPO₄ | 25 mM |
| KH₂PO₄ | 10 mM |
| Water | Remainder |

### Example 21

An aqueous germicidal solution having the concentrations listed in Table 19. was prepared and tested to determine its effectiveness at killing *Bacillus subtilis* spores. The tests were conducted at a pH of about 7.5, a temperature of 20°C, and an exposure time of 4 hours. The results indicated that the solution was effective to achieve a total kill of the spores in 4 hours.

**Table 19**

| Component | Concentration |
|---|---|
| Phthalaldehyde | 0.55% (w/v) |
| KF | 1000 mM |
| Benzotriazole | 1 mM |
| Water | Remainder |

### Example 22

An aqueous germicidal solution having the concentrations listed in Table 20 was prepared and tested to determine its effectiveness at killing *Bacillus subtilis* spores. The tests were conducted at a pH of about 7.5, a temperature of 20°C, and an exposure time of 4 hours. The results indicated that the solution was effective to achieve a total kill of the spores in 4 hours.

**Table 20**

| Component | Concentration |
|---|---|
| Phthalaldehyde | 0.55% (w/v) |
| KF | 1000mM |
| K₂HPO₄ | 25 mM |
| KH₂PO₄ | 10mM |
| EDTA·2Na | 5 mM |
| EDTA·4Na | 5 mM |
| Water | Remainder |

### Example 23

A germicidal solution may be prepared by dissolving the ingredients listed in Table 21 in about one liter of water. Then, the pH of the solution may be measured and sufficient hydrochloric acid or sodium hydroxide added to give a pH of about 7.2. The solution may be stored in an airtight pressurized container designed for an internal pressure of from 5 to 30 psi to help prevent escape of carbon dioxide.

**Table 21**

| Component | Concentration |
|---|---|
| Phthalaldehyde | 0.55% (w/v) |
| NaCl | 0-250 mM |
| NaHCO₃ | 250 mM |
| Na2HPO4·H2O | 250 mM |
| EDTA·2Na | 5 mM |
| EDTA·4Na | 5mM |
| Benzotriazole | 0-0.1 mM |
| Water | Remainder |

### Example 24

This prospective example demonstrates a first approach for preparing a solid composition according to Table 22. Fine particles of phthalaldehyde having either a nano-size or micron-size are prepared by grinding. Fine particles of the other ingredients were ground and sieved to obtain those particles having a size of 200-mesh or finer. In another prospective example all ingredients may be combined together and then ground to an appropriate particle size. The phthalaldehyde and the other ingredients were combined and mixed. Then, the mixed composition was placed in a mechanical press and pressed into a shaped solid. The shaped solid was sealed in an airtight laminated aluminum pouch.

**Table 22**

| Component | Amount |
|---|---|
| Phthalaldehyde | 4-6 grams |
| Na₂CO₃ | 25-55 grams |
| EDTA·4Na | 0-4 grams |
| EDTA (Free Acid) | 0-60 grams |
| NaH2PO4·H2O | 30-40 grams |
| Citric Acid | 0-20 grams |
| Benzotriazole | 0-0.05 grams |
| NaCl, Na₂SO₄, KF, or | combination 0-50 grams |
| Starch | 0-2 grams |

### Example 25

In this prospective example, the solid composition according to Table 22 may be prepared by dissolving all ingredients into a solution and then spray drying the solution to form a fine powder. The fine powder may be pressed and packaged as previously discussed.

### XI. GENERAL MATTERS

In the description above, for the purposes of explanation, numerous specific details are set forth in order to provide a thorough understanding of the embodiments of the invention. It will be apparent, however, to one skilled in the art that another embodiment may be practiced without some of these specific details. In other instances, well-known structures, devices, and techniques have been shown in block diagram form or without detail in order not to obscure the understanding of this description.

Many of the methods are described in their most basic form, but operations may be added to or deleted from any of the methods without departing from the basic scope of the invention. It will be apparent to those skilled in the art that many further modifications and adaptations may be made. The particular embodiments are not provided to limit the invention but to illustrate it. The scope of the invention is not to be determined by the specific examples provided above but only by the claims below.

It should also be appreciated that reference throughout this specification to "one embodiment" or "an embodiment" means that a particular feature may be included in the practice of the invention. Similarly, it should be appreciated that in the foregoing description of exemplary embodiments of the invention, various features are sometimes grouped together in a single embodiment, Figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

In the claims, any element that does not explicitly state "means for" performing a specified function, or "step for" performing a specified function, is not to be interpreted as a "means" or "step" clause as specified in 35 U.S.C. Section 112, Paragraph 6. In particular, the use of "step of" in the claims herein is not intended to invoke the provisions of 35 U.S.C. Section 112, Paragraph 6.

While the invention has been described in terms of several embodiments, those skilled in the art will recognize that the invention is not limited to the embodiments described, but may be practiced with modification and alteration within the spirit and scope of the appended claims. The description is thus to be regarded as illustrative instead of limiting.

## Claims

1. A solid composition comprising:
a solid having a water solubility greater than phthalaldehyde; and
phthalaldehyde diluted in the solid.

2. The solid composition of claim 1, wherein the solid comprises a salt.

3. The solid composition of claim 2, wherein the salt comprises an efficacy enhancing salt that is selected from the group consisting of a carbonate salt, a bicarbonate salt, a phosphate salt, a halide salt, and combinations thereof.

4. The solid composition of claim 1, further comprising nanoparticles containing the solid phthalaldehyde.

5. The solid composition of claim 4, further comprising the solid within the nanoparticles containing the phthalaldehyde to facilitate dissolution of the nanoparticles.

6. The solid composition of claim 1, further comprising a shaped solid having a predetermined size and shape containing the solid composition.

7. The solid composition of claim 1, further comprising a powder containing the solid composition, wherein the powder includes particles of the phthalaldehyde diluted in particles of the solid.

8. An apparatus comprising a water-resistant container having the solid composition of claim 1 contained therein.

9. A method of preparing a germicidal solution from the solid composition of claim 8, and killing microorganisms with the prepared composition, comprising:
opening the container;
removing the solid composition from the container;
combining the solid composition with a solvent;
dissolving the solid composition in the solvent to prepare the germicidal solution; and
killing the microorganisms by applying the solution to the microorganisms.

10. A kit to prepare a germicide solution comprising:
phthalaldehyde;
an enhancer for the phthalaldehyde;
at least two compartments to contain the phthalaldehyde and the enhancer.

11. The kit of claim 10, wherein the enhancer comprises an enhancer selected from the group consisting of a carbonate, a halide salt, and a phosphate.

12. The kit of claim 10, further comprising a solvent, wherein the solvent is in one of either the two compartments or a third compartment.

13. The kit of claim 10, further comprising solid phthalaldehyde of the phthalaldehyde.

14. The kit of claim 10, further comprising a first composition including the phthalaldehyde and a second composition, wherein the first composition has a pH that is less than 8, and wherein the second composition has a more alkaline pH than the first composition such that a combination of the first composition and the second composition has a pH that is greater than 8.

15. A method of preparing the germicidal solution using the kit of claim 10, the method comprising using an automated machine to autonomously combine the phthalaldehyde and the enhancer.

16. A germicidal solution preparation apparatus comprising:
a first port to receive a first solution preparation composition, the first solution preparation composition including a germicidally effective compound;
a second port to receive a second solution preparation composition, the second solution preparation composition including an enhancer for the germicidally effective compound;
a source of water;
germicidal solution preparation logic to control preparation of the germicidal solution from the first and the second compositions and the water.

17. The apparatus of claim 16, wherein the germicidally effective compound comprises phthalaldehyde.

18. The apparatus of claim 17, wherein the enhancer comprises an enhancer that is selected from the group consisting of a carbonate, a bicarbonate, a phosphate, an alkali metal halide, and combinations thereof.

19. The apparatus of claim 16, further comprising a heater to facilitate dissolution of the germicidally effective compound.

20. The apparatus of claim 19, wherein the heater is to melt the germicidally effective compound.

21. The apparatus of claim 16, further comprising a germicidal solution interrogation system of the apparatus to determine a concentration of the germicidally effective compound.

22. The apparatus of claim 16, further comprising a feedback control mechanism to provide the first solution preparation composition from the first port.

23. The apparatus of claim 16, further comprising a feedback control mechanism to provide the second solution preparation composition from the second port.
